# EUROPEAN PATENT SPECIFICATION

(11) **EP 3 490 396 B1**
(45) Date of publication and mention of the grant of the patent: **03.07.2024**
(21) Application number: 17758288.9
(22) Date of filing: 26.07.2017
(51) Int. Cl.: A24F 40/46, A24F 40/485, A24F 40/10

(54) **AEROSOL DELIVERY DEVICE INCLUDING A SELECTOR AND RELATED METHOD**
AEROSOLFREISETZUNGSVORRICHTUNG MIT EINER AUSWAHLVORRICHTUNG UND ZUGEHÖRIGES VERFAHREN
DISPOSITIF DE DISTRIBUTION D'AÉROSOL COMPRENANT UN SÉLECTEUR ET PROCÉDÉ ASSOCIÉ

(30) Priority: 28.07.2016 US 201615222615
(43) Date of publication of application: 05.06.2019
(60) Divisional application: 24169972.7
(73) Proprietor: RAI Strategic Holdings, Inc., Winston-Salem, NC 27101 (US)
(72) Inventor: WATSON, Nicholas H., Westfield, NC 27053 (US); DAVIS, Michael F., Clemmons, NC 27012 (US); SEARS, Stephen Benson, Siler City, NC 27344 (US)
(74) Representative: Hoeger, Stellrecht & Partner Patentanwälte mbB
(86) International application number: PCT/IB2017/054549
(87) International publication number: WO 2018/020444

(56) References cited:
- EP-A1- 2 989 912
- WO-A1-2016/090426
- US-A1- 2016 206 001
- US-B2- 8 881 737

## Description

### BACKGROUND

### Field of the Disclosure

The present disclosure relates to aerosol delivery devices such as electronic cigarettes, and more particularly to aerosol delivery devices including an atomizer. The atomizer may be configured to heat an aerosol precursor composition, which may be made or derived from tobacco or otherwise incorporate tobacco, to form an inhalable substance for human consumption.

### Description of Related Art

WO 2016/090426 A1 discloses a cartridge for an electronic cigarette or vaporizer including one or more reservoirs, first and second heating elements, and an electrical circuit. The reservoirs hold liquid to be vaporized. The first and second heating elements are configured to vaporize the liquid. The electrical circuit selectively activates the first and second heating elements in different modes.

EP 2 989 912 A1 discloses an electronic smoking device including a mouthpiece, a heating element and a body portion which contains a liquid reservoir. The heating element is adapted to vaporize liquid supplied from the reservoir to generate an aerosol. A flow path extends from the heating element to the mouthpiece and receives the generated aerosol. Two or more flavor-carrying units are permeable to vaporize liquid and carry flavored material. The flavor-carrying units are selectively and individually exposed to the flow path creating a flavored aerosol which can be inhaled by a user.

Many devices have been proposed through the years as improvements upon, or alternatives to, smoking products that require combusting tobacco for use. Many of those devices purportedly have been designed to provide the sensations associated with cigarette, cigar, or pipe smoking, but without delivering considerable quantities of incomplete combustion and pyrolysis products that result from the burning of tobacco. To this end, there have been proposed numerous alternative smoking products, flavor generators, and medicinal inhalers that utilize electrical energy to vaporize or heat a volatile material, or attempt to provide the sensations of cigarette, cigar, or pipe smoking without burning tobacco to a significant degree. See, for example, the various alternative smoking articles, aerosol delivery devices and heat generating sources set forth in the background art described in U.S. Pat. No. 8,881,737 to Collett et al., U.S. Pat. App. Pub. No. 2013/0255702 to Griffith Jr. et al., U.S. Pat. App. Pub. No. 2014/0000638 to Sebastian et al., U.S. Pat. App. Pub. No. 2014/0096781 to Sears et al., U.S. Pat. App. Pub. No. 2014/0096782 to Ampolini et al., and U.S. Pat. App. Pub. No. 2015/0059780 to Davis et al. See also, for example, the various embodiments of products and heating configurations described in the background sections of U.S. Pat. Nos. 5,388,594 to Counts et al. and 8,079,371 to Robinson et al.

However, it may be desirable to provide aerosol delivery devices with additional user control or customization. Thus, advances with respect to aerosol delivery device functionality may be desirable.

### BRIEF SUMMARY OF THE DISCLOSURE

The present disclosure relates to assembly of cartridges for aerosol delivery devices configured to produce aerosol and which aerosol delivery devices, in some embodiments, may be referred to as electronic cigarettes.

In one aspect an aerosol delivery device is provided. The aerosol delivery device includes at least one atomizer configured to produce a vapor from an aerosol precursor composition. Further, the aerosol delivery device includes an additive selector configured to provide for selection of one or more of a plurality of additives added to the vapor.

The additive selector includes a bed of solids. The bed of solids may be positioned downstream of the at least one atomizer in terms of an airflow path. The bed of solids may include a plurality of compartments separated by one or more partitions. The aerosol delivery device may further include a flow director. The additive selector may be configured to selectively align the flow director with one or more of the compartments. The additive selector may further include one or more additive heating elements configured to selectively heat one or more portions of the bed of solids. The bed of solids may include a plurality of flavor-laden plastic solids.

In some embodiments the at least one atomizer may include a Venturi nozzle. The additive selector may include a plurality of channels each configured to be in fluid communication with one of a plurality of additive reservoirs and selectively configurable to be in fluid communication with the Venturi nozzle. The additive selector may include at least one crystal oscillator. The at least one atomizer may include a plurality of atomizers each configured to be in fluid communication with a respective one of a plurality of reservoirs. The aerosol delivery device may further include an atomizer selector configured to provide for selection of one or more of the atomizers to which electrical current is directed to produce the vapor therefrom and alter a position of the atomizers with respect to an airflow path through the aerosol delivery device.

In an additional aspect a method for vapor production with an aerosol delivery device is provided. The method includes providing for selection of one or more of a plurality of additives. Further, the method includes producing a vapor with at least one atomizer from an aerosol precursor composition. The method additionally includes adding the one or more additives selected to the vapor.

The method further includes forming the one or more additives from a bed of solids. Providing for selection of one or more of the additives may include providing for selective alignment of a Venturi nozzle with one or more channels respectively in fluid communication with one of a plurality of additive reservoirs. Adding the one or more additives selected to the vapor may include activating at least one crystal oscillator. Producing the vapor with at least one atomizer may include providing for selection of one or more of a plurality of atomizers. The method may further include altering a position of the atomizers with respect to an airflow path through the aerosol delivery device and directing electrical current to the one or more of the atomizers selected to produce a vapor.

The present disclosure thus includes, without limitation, the following:
An aerosol delivery device, comprising: at least one atomizer configured to produce a vapor from an aerosol precursor composition; and an additive selector configured to provide for selection of one or more of a plurality of additives added to the vapor, wherein the additive selector comprises: a bed of solids, and one or more additive heating elements configured to selectively heat one or more portions of the bed of solids.

The device of above, wherein the bed of solids is positioned downstream of the at least one atomizer in terms of an airflow path.

The device of above, wherein the bed of solids comprises a plurality of compartments separated by one or more partitions.

The device of above, further comprising a flow director, the additive selector being configured to selectively align the flow director with one or more of the compartments.

The device of above, wherein the bed of solids comprises a plurality of flavor-laden plastic solids.

The device of above, wherein the at least one atomizer comprises a Venturi nozzle, and wherein the additive selector comprises a plurality of channels each configured to be in fluid communication with one of a plurality of additive reservoirs and selectively configurable to be in fluid communication with the Venturi nozzle.

The device of above, wherein the additive selector comprises at least one crystal oscillator.

The device of above, wherein the at least one atomizer includes a plurality of atomizers each configured to be in fluid communication with a respective one of a plurality of reservoirs.

The device of above, further comprising an atomizer selector configured to: provide for selection of one or more of the atomizers to which electrical current is directed to produce the vapor therefrom; and alter a position of the atomizers with respect to an airflow path through the aerosol delivery device.

A method for vapor production with an aerosol delivery device, the method comprising: forming one or more of a plurality of additives from a bed of solids; providing for selection of the one or more of a plurality of additives; selectively heating one or more portions of the bed of solids to release the selected one or more of the plurality of additives from the bed of solids; producing a vapor with at least one atomizer from an aerosol precursor composition; and adding the released one or more of the plurality of additives selected to the vapor.

The method of above, wherein providing for selection of one or more of the additives comprises providing for selective alignment of a Venturi nozzle with one or more channels respectively in fluid communication with one of a plurality of additive reservoirs.

The method of above, wherein adding the one or more additives selected to the vapor comprises activating at least one crystal oscillator.

The method of above, wherein producing the vapor with at least one atomizer comprises providing for selection of one or more of a plurality of atomizers.

The method of above, further comprising altering a position of the atomizers with respect to an airflow path through the aerosol delivery device; and directing electrical current to the one or more of the atomizers selected to produce a vapor.

These and other features, aspects, and advantages of the present disclosure will be apparent from a reading of the following detailed description together with the accompanying drawings, which are briefly described below.

### BRIEF DESCRIPTION OF THE FIGURES

Having thus described the disclosure in the foregoing general terms, reference will now be made to the accompanying drawings, which are not necessarily drawn to scale, and wherein:
FIG. 1 illustrates an aerosol delivery device comprising a cartridge and a control body in an assembled configuration according to an example embodiment of the present disclosure;
FIG. 2 illustrates the control body of FIG. 1 in an exploded configuration according to an example embodiment of the present disclosure;
FIG. 3 illustrates the cartridge of FIG. 1 in an exploded configuration according to an example embodiment of the present disclosure;
FIG. 4 schematically illustrates an aerosol delivery device including an atomizer selector, one or more atomizers, and one or more reservoirs according to an example embodiment of the present disclosure;
FIG. 5 schematically illustrates an embodiment of the aerosol delivery device of FIG. 4 comprising a plurality of atomizers and a plurality of reservoirs;
FIG. 6 illustrates a perspective view of an atomizer selector including a plurality of guide tracks and first and second atomizers in an electrically disconnected configuration according to an example embodiment of the present disclosure;
FIG. 7 illustrates a top view of the atomizer selector of FIG. 6 wherein the first atomizer is electrically connected;
FIG. 8 illustrates a top view of the atomizer selector of FIG. 6 wherein the second atomizer is electrically connected;
FIG. 9 illustrates a top view of an atomizer selector including a plurality of guide tracks and first and second atomizers each in an electrically connected configuration according to an example embodiment of the present disclosure;
FIG. 10 illustrates a top view of the atomizer selector of FIG. 9 wherein only the first atomizer is electrically connected;
FIG. 11 illustrates a top view of the atomizer selector of FIG. 9 wherein only the second atomizer is electrically connected;
FIG. 12 schematically illustrates an atomizer selector including a switch electrically connected to a first atomizer according to an example embodiment of the present disclosure;
FIG. 13 schematically illustrates the atomizer selector of FIG. 12 wherein the switch is electrically connected to a second atomizer according to an example embodiment of the present disclosure;
FIG. 14 schematically illustrates the atomizer selector of FIG. 14 wherein the switch is electrically connected to the first and second atomizers according to an example embodiment of the present disclosure;
FIG. 15 schematically illustrates an atomizer selector including a switch electrically connected to first and second atomizers and a valve directing airflow to the first and second atomizers according to an example embodiment of the present disclosure;
FIG. 16 schematically illustrates the atomizer selector of FIG. 15 wherein the switch is electrically coupled to the first atomizer and the valve directs airflow to the first atomizer;
FIG. 17 schematically illustrates the atomizer selector of FIG. 15 wherein the switch is electrically coupled to the second atomizer and the valve directs airflow to the second atomizer;
FIG. 18 schematically illustrates an atomizer selector including a switch electrically connected to first and second atomizers and first and second valves in first and second flow directors to allow airflow to the first and second atomizers according to an example embodiment of the present disclosure;
FIG. 19 schematically illustrates the atomizer selector of FIG. 18 wherein the switch is electrically connected to the first atomizer, the first valve is open, and the second valve is closed;
FIG. 20 schematically illustrates the atomizer selector of FIG. 18 wherein the switch is electrically connected to the second atomizer, the second valve is open, and the first valve is closed;
FIG. 21 schematically illustrates an aerosol delivery device including an additive selector, one or more atomizers, and one or more reservoirs according to the invention;
FIG. 22 schematically illustrates a configuration of the one or more atomizers and the additive selector of the aerosol delivery device of FIG. 21 relative to an airflow therethrough;
FIG. 23 illustrates a top view of a flow director and an additive selector including a bed of solids, wherein the flow director is aligned with a first compartment according to the invention;
FIG. 24 illustrates a top view of the flow director and the additive selector of FIG. 23, wherein the flow director is aligned with second and third compartments according to an example embodiment of the invention;
FIG. 25 illustrates a top view of a flow director and an additive selector including a bed of solids including a plurality of additive heating elements, wherein the compartments are arranged perpendicular to the airflow therethrough according to an example embodiment of the invention;
FIG. 26 illustrates a side view of an additive selector including a bed of solids including a plurality of additive heating elements, wherein the compartments are arranged in-line with the airflow therethrough according to an example embodiment of the invention;
FIG. 27 illustrates a side view of an additive selector including a plurality of bubble jet heads according to an example embodiment of the present disclosure;
FIG. 28 illustrates a side view of an aerosol delivery device including a Venturi nozzle wherein a first channel of an additive selector is in fluid communication with the Venturi nozzle according to an example embodiment of the present disclosure;
FIG. 29 illustrates a side view of the aerosol delivery device of FIG. 28 wherein a second channel of an additive selector is in fluid communication with the Venturi nozzle according to an example embodiment of the present disclosure;
FIG. 30 illustrates a side view of an additive selector including a crystal oscillator in a decoupled configuration according to an example embodiment of the present disclosure;
FIG. 31 illustrates a side view of the additive selector of FIG. 30 wherein the crystal oscillator is in contact with a first channel according to an example embodiment of the present disclosure;
FIG. 32 schematically illustrates a method for vapor production with an aerosol delivery device including altering a position of atomizers with respect to an airflow path through the aerosol delivery device according to an example embodiment of the present disclosure; and
FIG. 33 schematically illustrates a method for vapor production with an aerosol delivery device including providing for selection of one or more of a plurality of additives according to an example embodiment of the invention.

### DETAILED DESCRIPTION OF PREFERRED EMBODIMENTS

The present disclosure will now be described more fully hereinafter with reference to exemplary embodiments thereof. These exemplary embodiments are described so that this disclosure will be thorough and complete, and will fully convey the scope of the disclosure to those skilled in the art. Indeed, the disclosure may be embodied in many different forms and should not be construed as limited to the embodiments set forth herein; rather, these embodiments are provided so that this disclosure will satisfy applicable legal requirements. The scope of the invention is defined by the appended claims.

As used in the specification, and in the appended claims, the singular forms "a", "an", "the", include plural variations unless the context clearly dictates otherwise.

The present disclosure provides descriptions of aerosol delivery devices. The aerosol delivery devices may use electrical energy to heat a material (preferably without combusting the material to any significant degree) to form an inhalable substance; such articles most preferably being sufficiently compact to be considered "hand-held" devices. An aerosol delivery device may provide some or all of the sensations (e.g., inhalation and exhalation rituals, types of tastes or flavors, organoleptic effects, physical feel, use rituals, visual cues such as those provided by visible aerosol, and the like) of smoking a cigarette, cigar, or pipe, without any substantial degree of combustion of any component of that article or device. The aerosol delivery device may not produce smoke in the sense of the aerosol resulting from by-products of combustion or pyrolysis of tobacco, but rather, that the article or device most preferably yields vapors (including vapors within aerosols that can be considered to be visible aerosols that might be considered to be described as smoke-like) resulting from volatilization or vaporization of certain components of the article or device, although in other embodiments the aerosol may not be visible. In highly preferred embodiments, aerosol delivery devices may incorporate tobacco and/or components derived from tobacco. As such, the aerosol delivery device can be characterized as an electronic smoking article such as an electronic cigarette or "e-cigarette."

While the systems are generally described herein in terms of embodiments associated with aerosol delivery devices such as so-called "e-cigarettes," it should be understood that the mechanisms, components, features, and methods may be embodied in many different forms and associated with a variety of articles. For example, the description provided herein may be employed in conjunction with embodiments of traditional smoking articles (e.g., cigarettes, cigars, pipes, etc.), heat-not-burn cigarettes, and related packaging for any of the products disclosed herein. Accordingly, it should be understood that the description of the mechanisms, components, features, and methods disclosed herein are discussed in terms of embodiments relating to aerosol delivery devices by way of example only, and may be embodied and used in various other products and methods.

Aerosol delivery devices of the present disclosure also can be characterized as being vapor-producing articles or medicament delivery articles. Thus, such articles or devices can be adapted so as to provide one or more substances (e.g., flavors and/or pharmaceutical active ingredients) in an inhalable form or state. For example, inhalable substances can be substantially in the form of a vapor (i.e., a substance that is in the gas phase at a temperature lower than its critical point). Alternatively, inhalable substances can be in the form of an aerosol (i.e., a suspension of fine solid particles or liquid droplets in a gas). For purposes of simplicity, the term "aerosol" as used herein is meant to include vapors, gases and aerosols of a form or type suitable for human inhalation, whether or not visible, and whether or not of a form that might be considered to be smoke-like.

In use, aerosol delivery devices of the present disclosure may be subjected to many of the physical actions employed by an individual in using a traditional type of smoking article (e.g., a cigarette, cigar or pipe that is employed by lighting and inhaling tobacco). For example, the user of an aerosol delivery device of the present disclosure can hold that article much like a traditional type of smoking article, draw on one end of that article for inhalation of aerosol produced by that article, take puffs at selected intervals of time, etc.

Aerosol delivery devices of the present disclosure generally include a number of components provided within an outer shell or body. The overall design of the outer shell or body can vary, and the format or configuration of the outer body that can define the overall size and shape of the aerosol delivery device can vary. Typically, an elongated body resembling the shape of a cigarette or cigar can be a formed from a single, unitary shell; or the elongated body can be formed of two or more separable pieces. For example, an aerosol delivery device can comprise an elongated shell or body that can be substantially tubular in shape and, as such, resemble the shape of a conventional cigarette or cigar. However, various other shapes and configurations may be employed in other embodiments (e.g., rectangular or fob-shaped).

In one embodiment, all of the components of the aerosol delivery device are contained within one outer body or shell. Alternatively, an aerosol delivery device can comprise two or more shells that are joined and are separable. For example, an aerosol delivery device can possess at one end a control body comprising a shell containing one or more reusable components (e.g., a rechargeable battery and various electronics for controlling the operation of that article), and at the other end and removably attached thereto a shell containing a disposable portion (e.g., a disposable flavor-containing cartridge). More specific formats, configurations and arrangements of components within the single shell type of unit or within a multi-piece separable shell type of unit will be evident in light of the further disclosure provided herein. Additionally, various aerosol delivery device designs and component arrangements can be appreciated upon consideration of the commercially available electronic aerosol delivery devices.

Aerosol delivery devices of the present disclosure most preferably comprise some combination of a power source (i.e., an electrical power source), at least one control component (e.g., means for actuating, controlling, regulating and/or ceasing power for heat generation, such as by controlling electrical current flow from the power source to other components of the aerosol delivery device), a heater or heat generation component (e.g., an electrical resistance heating element or component commonly referred to as part of an "atomizer"), and an aerosol precursor composition (e.g., commonly a liquid capable of yielding an aerosol upon application of sufficient heat, such as ingredients commonly referred to as "smoke juice," "e-liquid" and "e-juice"), and a mouthend region or tip for allowing draw upon the aerosol delivery device for aerosol inhalation (e.g., a defined air flow path through the article such that aerosol generated can be withdrawn therefrom upon draw).

Alignment of the components within the aerosol delivery device of the present disclosure can vary. In specific embodiments, the aerosol precursor composition can be located near an end of the aerosol delivery device which may be configured to be positioned proximal to the mouth of a user so as to maximize aerosol delivery to the user. Other configurations, however, are not excluded. Generally, the heating element can be positioned sufficiently near the aerosol precursor composition so that heat from the heating element can volatilize the aerosol precursor (as well as one or more flavorants, medicaments, or the like that may likewise be provided for delivery to a user) and form an aerosol for delivery to the user. When the heating element heats the aerosol precursor composition, an aerosol is formed, released, or generated in a physical form suitable for inhalation by a consumer. It should be noted that the foregoing terms are meant to be interchangeable such that reference to release, releasing, releases, or released includes form or generate, forming or generating, forms or generates, and formed or generated. Specifically, an inhalable substance is released in the form of a vapor or aerosol or mixture thereof, wherein such terms are also interchangeably used herein except where otherwise specified.

As noted above, the aerosol delivery device may incorporate a battery or other electrical power source (e.g., a capacitor) to provide current flow sufficient to provide various functionalities to the aerosol delivery device, such as powering of a heater, powering of control systems, powering of indicators, and the like. The power source can take on various embodiments. Preferably, the power source is able to deliver sufficient power to rapidly heat the heating element to provide for aerosol formation and power the aerosol delivery device through use for a desired duration of time. The power source preferably is sized to fit conveniently within the aerosol delivery device so that the aerosol delivery device can be easily handled. Additionally, a preferred power source is of a sufficiently light weight to not detract from a desirable smoking experience.

More specific formats, configurations and arrangements of components within the aerosol delivery device of the present disclosure will be evident in light of the further disclosure provided hereinafter. Additionally, the selection of various aerosol delivery device components can be appreciated upon consideration of the commercially available electronic aerosol delivery devices. Further, the arrangement of the components within the aerosol delivery device can also be appreciated upon consideration of the commercially available electronic aerosol delivery devices. Examples of commercially available products, for which the components thereof, methods of operation thereof, materials included therein, and/or other attributes thereof may be included in the devices of the present disclosure have been marketed as ACCORD^{®} by Philip Morris Incorporated; ALPHA^{™}, JOYE 510^{™} and M4^{™} by InnoVapor LLC; CIRRUS^{™} and FLING^{™} by White Cloud Cigarettes; BLU^{™} by Lorillard Technologies, Inc.; COHITA^{™}, COLIBRI^{™}, ELITE CLASSIC^{™}, MAGNUM^{™}, PHANTOM^{™} and SENSE^{™} by Epuffer^{®} International Inc.; DUOPRO^{™}, STORM^{™} and VAPORKING^{®} by Electronic Cigarettes, Inc.; EGAR^{™} by Egar Australia; eGo-C^{™} and eGo-T^{™} by Joyetech; ELUSION^{™} by Elusion UK Ltd; EONSMOKE^{®} by Eonsmoke LLC; FIN^{™} by FIN Branding Group, LLC; SMOKE^{®} by Green Smoke Inc. USA; GREENARETTE^{™} by Greenarette LLC; HALLIGAN^{™}, HENDU^{™}, JET^{™}, MAXXQ^{™}, PINK^{™} and PITBULL^{™} by Smoke Stik^{®}; HEATBAR^{™} by Philip Morris International, Inc.; HYDRO IMPERIAL^{™} and EXE^{™} from Crown7; LOGIC^{™} and THE CUBAN^{™} by LOGIC Technology; LUCI^{®} by Luciano Smokes Inc.; METRO^{®} by Nicotek, LLC; NJOY^{®} and ONEJOY^{™} by Sottera, Inc.; NO. 7^{™} by SS Choice LLC; PREMIUM ELECTRONIC CIGARETTE^{™} by PremiumEstore LLC; RAPP E-MYSTICK^{™} by Ruyan America, Inc.; RED DRAGON^{™} by Red Dragon Products, LLC; RUYAN^{®} by Ruyan Group (Holdings) Ltd.; SF^{®} by Smoker Friendly International, LLC; GREEN SMART SMOKER^{®} by The Smart Smoking Electronic Cigarette Company Ltd.; SMOKE ASSIST^{®} by Coastline Products LLC; SMOKING EVERYWHERE^{®} by Smoking Everywhere, Inc.; V2CIGS^{™} by VMR Products LLC; VAPOR NINE^{™} by VaporNine LLC; VAPOR4LIFE^{®} by Vapor 4 Life, Inc.; VEPPO^{™} by E-CigaretteDirect, LLC; AVIGO, VUSE, VUSE CONNECT, VUSE FOB, VUSE HYBRID, ALTO, ALTO+, MODO, CIRO, FOX + FOG, AND SOLO+ by R. J. Reynolds Vapor Company; MISTIC MENTHOL by Mistic Ecigs; and VYPE by CN Creative Ltd. Yet other electrically powered aerosol delivery devices, and in particular those devices that have been characterized as so-called electronic cigarettes, have been marketed under the tradenames COOLER VISIONS^{™}; DIRECT E-CIG^{™}; DRAGONFLY^{™}; EMIST^{™}; EVERSMOKE^{™}; GAMUCCI^{®}; HYBRID FLAME^{™}; KNIGHT STICKS^{™}; ROYAL BLUES^{™}; SMOKETIP^{®}; SOUTH BEACH SMOKE^{™}.

Additional manufacturers, designers, and/or assignees of components and related technologies that may be employed in the aerosol delivery device of the present disclosure include Shenzhen Jieshibo Technology of Shenzhen, China; Shenzhen First Union Technology of Shenzhen City, China; Safe Cig of Los Angeles, CA; Janty Asia Company of the Philippines; Joyetech Changzhou Electronics of Shenzhen, China; SIS Resources; B2B International Holdings of Dover, DE; Evolv LLC of OH; Montrade of Bologna, Italy; Shenzhen Bauway Technology of Shenzhen, China; Global Vapor Trademarks Inc. of Pompano Beach, FL; Vapor Corp. of Fort Lauderdale, FL; Nemtra GMBH of Raschau-Markersbach, Germany, Perrigo L. Co. of Allegan, MI; Needs Co., Ltd.; Smokefree Innotec of Las Vegas, NV; McNeil AB of Helsingborg, Sweden; Chong Corp; Alexza Pharmaceuticals of Mountain View, CA; BLEC, LLC of Charlotte, NC; Gaitrend Sari of Rohrbach-lès-Bitche, France; FeelLife Bioscience International of Shenzhen, China; Vishay Electronic BMGH of Selb, Germany; Shenzhen Smaco Technology Ltd. of Shenzhen, China; Vapor Systems International of Boca Raton, FL; Exonoid Medical Devices of Israel; Shenzhen Nowotech Electronic of Shenzhen, China; Minilogic Device Corporation of Hong Kong, China; Shenzhen Kontle Electronics of Shenzhen, China, and Fuma International, LLC of Medina, OH, 21st Century Smoke of Beloit, WI, and Kimree Holdings (HK) Co. Limited of Hong Kong, China.

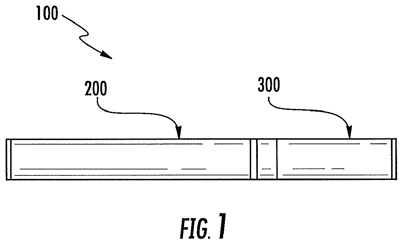

One example embodiment of an aerosol delivery device 100 is illustrated in FIG. 1. In particular, FIG. 1 illustrates an aerosol delivery device 100 including a control body 200 and a cartridge 300. The control body 200 and the cartridge 300 can be permanently or detachably aligned in a functioning relationship. Various mechanisms may connect the cartridge 300 to the control body 200 to result in a threaded engagement, a press-fit engagement, an interference fit, a magnetic engagement, or the like. The aerosol delivery device 100 may be substantially rod-like, substantially tubular shaped, or substantially cylindrically shaped in some embodiments when the cartridge 300 and the control body 200 are in an assembled configuration. However, as noted above, various other configurations such as rectangular or fob-shaped may be employed in other embodiments. Further, although the aerosol delivery devices are generally described herein as resembling the size and shape of a traditional smoking article, in other embodiments differing configurations and larger capacity reservoirs, which may be referred to as "tanks," may be employed.

In specific embodiments, one or both of the cartridge 300 and the control body 200 may be referred to as being disposable or as being reusable. For example, the control body 200 may have a replaceable battery or a rechargeable battery and/or capacitor and thus may be combined with any type of recharging technology, including connection to a typical alternating current electrical outlet, connection to a car charger (i.e., cigarette lighter receptacle), and connection to a computer, such as through a universal serial bus (USB) cable. Further, in some embodiments the cartridge 300 may comprise a single-use cartridge, as disclosed in U.S. Pat. No. 8,910,639 to Chang et al.

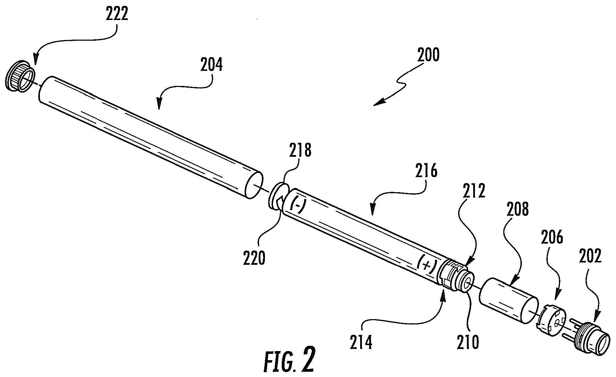

FIG. 2 illustrates an exploded view of the control body 200 of the aerosol delivery device 100 (see, FIG. 1) according to an example embodiment of the present disclosure. As illustrated, the control body 200 may comprise a coupler 202, an outer body 204, a sealing member 206, an adhesive member 208 (e.g., KAPTON^{®} tape), a flow sensor 210 (e.g., a puff sensor or pressure switch), a control component 212, a spacer 214, an electrical power source 216 (e.g., a capacitor and/or a battery, which may be rechargeable), a circuit board with an indicator 218 (e.g., a light emitting diode (LED)), a connector circuit 220, and an end cap 222. Examples of electrical power sources are described in U.S. Pat. App. Pub. No. 2010/0028766 by Peckerar et al.

With respect to the flow sensor 210, representative current regulating components and other current controlling components including various microcontrollers, sensors, and switches for aerosol delivery devices are described in U.S. Pat. No. 4,735,217 to Gerth et al., U.S. Pat. Nos. 4,922,901, 4,947,874, and 4,947,875, all to Brooks et al., U.S. Pat. No. 5,372,148 to McCafferty et al., U.S. Pat. No. 6,040,560 to Fleischhauer et al., U.S. Pat. No. 7,040,314 to Nguyen et al., and U.S. Pat. No. 8,205,622 to Pan. Reference also is made to the control schemes described in U.S. App. Pub. No. 2014/0270727 to Ampolini et al.

In one embodiment the indicator 218 may comprise one or more light emitting diodes. The indicator 218 can be in communication with the control component 212 through the connector circuit 220 and be illuminated, for example, during a user drawing on a cartridge coupled to the coupler 202, as detected by the flow sensor 210. The end cap 222 may be adapted to make visible the illumination provided thereunder by the indicator 218. Accordingly, the indicator 218 may be illuminated during use of the aerosol delivery device 100 to simulate the lit end of a smoking article. However, in other embodiments the indicator 218 can be provided in varying numbers and can take on different shapes and can even be an opening in the outer body (such as for release of sound when such indicators are present).

Still further components can be utilized in the aerosol delivery device of the present disclosure. For example, U.S. Pat. No. 5,154,192 to Sprinkel et al. discloses indicators for smoking articles; U.S. Pat. No. 5,261,424 to Sprinkel, Jr. discloses piezoelectric sensors that can be associated with the mouth-end of a device to detect user lip activity associated with taking a draw and then trigger heating of a heating device; U.S. Pat. No. 5,372,148 to McCafferty et al. discloses a puff sensor for controlling energy flow into a heating load array in response to pressure drop through a mouthpiece; U.S. Pat. No. 5,967,148 to Harris et al. discloses receptacles in a smoking device that include an identifier that detects a non-uniformity in infrared transmissivity of an inserted component and a controller that executes a detection routine as the component is inserted into the receptacle; U.S. Pat. No. 6,040,560 to Fleischhauer et al. describes a defined executable power cycle with multiple differential phases; U.S. Pat. No. 5,934,289 to Watkins et al. discloses photonic-optronic components; U.S. Pat. No. 5,954,979 to Counts et al. discloses means for altering draw resistance through a smoking device; U.S. Pat. No. 6,803,545 to Blake et al. discloses specific battery configurations for use in smoking devices; U.S. Pat. No. 7,293,565 to Griffen et al. discloses various charging systems for use with smoking devices; U.S. Pat. No. 8,402,976 to Fernando et al. discloses computer interfacing means for smoking devices to facilitate charging and allow computer control of the device; U.S. Pat. No. 8,689,804 to Fernando et al. discloses identification systems for smoking devices; and WO 2010/003480 by Flick discloses a fluid flow sensing system indicative of a puff in an aerosol generating system. Further examples of components related to electronic aerosol delivery articles and disclosing materials or components that may be used in the present article include U.S. Pat. No. 4,735,217 to Gerth et al.; U.S. Pat. No. 5,249,586 to Morgan et al.; U.S. Pat. No. 5,666,977 to Higgins et al.; U.S. Pat. No. 6,053,176 to Adams et al.; U.S. 6,164,287 to White; U.S. Pat No. 6,196,218 to Voges; U.S. Pat. No. 6,810,883 to Felter et al.; U.S. Pat. No. 6,854,461 to Nichols; U.S. Pat. No. 7,832,410 to Hon; U.S. Pat. No. 7,513,253 to Kobayashi; U.S. Pat. No. 7,896,006 to Hamano; U.S. Pat. No. 6,772,756 to Shayan; U.S. Pat. No. 8,156,944 and 8,375,957 to Hon; U.S. Pat. No. 8,794,231 to Thorens et al.; U.S. Pat. No. 8,851,083 to Oglesby et al.; U.S. Pat. No. 8,915,254 and 8,925,555 to Monsees et al.; and U.S. Pat. No. 9,220,302 to DePiano et al.; U.S. Pat. App. Pub. Nos. 2006/0196518 and 2009/0188490 to Hon; U.S. Pat. App. Pub. No. 2010/0024834 to Oglesby et al.; U.S. Pat. App. Pub. No. 2010/0307518 to Wang; WO 2010/091593 to Hon; and WO 2013/089551 to Foo. A variety of the materials disclosed by the foregoing documents may be incorporated into the present devices in various embodiments.

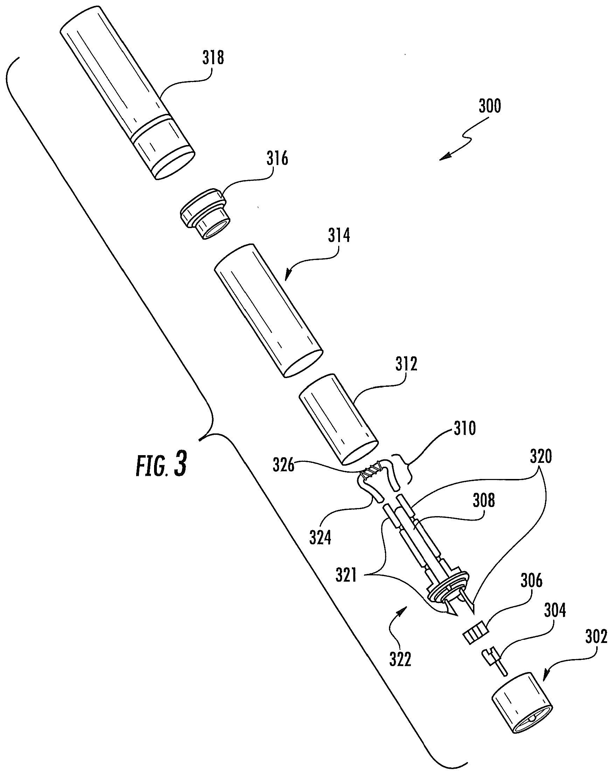

FIG. 3 illustrates the cartridge 300 of the aerosol delivery device 100 (see, FIG. 1) in an exploded configuration. As illustrated, the cartridge 300 may comprise a base 302, a control component terminal 304, an electronic control component 306, a flow director 308, an atomizer 310, a reservoir 312 (e.g., a reservoir substrate), an outer body 314, a mouthpiece 316, a label 318, and first and second heating terminals 320, 321 according to an example embodiment of the present disclosure.

In some embodiments the first and second heating terminals 320, 321 may be embedded in, or otherwise coupled to, the flow director 308. For example, the first and second heating terminals 320, 321 may be insert molded in the flow director 308. Accordingly, the flow director 308 and the first and second heating terminals are collectively referred to herein as a flow director assembly 322. Additional description with respect to the first and second heating terminals 320, 321 and the flow director 308 is provided in U.S. Pat. Pub. No. 2015/0335071 to Brinkley et al.

The atomizer 310 may comprise a liquid transport element 324 and a heating element 326. The cartridge may additionally include a base shipping plug engaged with the base and/or a mouthpiece shipping plug engaged with the mouthpiece in order to protect the base and the mouthpiece and prevent entry of contaminants therein prior to use as disclosed, for example, in U.S. Pat. No. 9,220,302 to Depiano et al.

The base 302 may be coupled to a first end of the outer body 314 and the mouthpiece 316 may be coupled to an opposing second end of the outer body to substantially or fully enclose other components of the cartridge 300 therein. For example, the control component terminal 304, the electronic control component 306, the flow director 308, the atomizer 310, and the reservoir 312 may be substantially or entirely retained within the outer body 314. The label 318 may at least partially surround the outer body 314, and optionally the base 302, and include information such as a product identifier thereon. The base 302 may be configured to engage the coupler 202 of the control body 200 (see, e.g., FIG. 2). In some embodiments the base 302 may comprise anti-rotation features that substantially prevent relative rotation between the cartridge and the control body as disclosed in U.S. Pat. App. Pub. No. 2014/0261495 to Novak et al.

The reservoir 312 may be configured to hold an aerosol precursor composition. Representative types of aerosol precursor components and formulations are also set forth and characterized in U.S. Pat. Nos. 7,726,320 to Robinson et al., 8,881,737 to Collett et al., and 9,254,002 to Chong et al.; and U.S. Pat. Pub. Nos. 2013/0008457 to Zheng et al.; 2015/0020823 to Lipowicz et al.; and 2015/0020830 to Koller, as well as WO 2014/182736 to Bowen et al. Other aerosol precursors that may be employed include the aerosol precursors that have been incorporated in the VUSE^{®} product by R. J. Reynolds Vapor Company, the BLU product by Lorillard Technologies, the MISTIC MENTHOL product by Mistic Ecigs, and the VYPE product by CN Creative Ltd. Also desirable are the so-called "smoke juices" for electronic cigarettes that have been available from Johnson Creek Enterprises LLC. Embodiments of effervescent materials can be used with the aerosol precursor, and are described, by way of example, in U.S. Pat. App. Pub. No. 2012/0055494 to Hunt et al. Further, the use of effervescent materials is described, for example, in U.S. Pat. No. 4,639,368 to Niazi et al.; U.S. Pat. No. 5,178,878 to Wehling et al.; U.S. Pat. No. 5,223,264 to Wehling et al.; U.S. Pat. No. 6,974,590 to Pather et al.; U.S. Pat. No. 7,381,667 to Bergquist et al.; U.S. Pat. No. 8,424,541 to Crawford et al; and U.S. Pat. No. 8,627,828 to Strickland et al.; as well as US Pat. Pub. Nos. 2010/0018539 to Brinkley et al. and 2010/0170522 to Sun et al.; and PCT WO 97/06786 to Johnson et al. Additional description with respect to embodiments of aerosol precursor compositions, including description of tobacco or components derived from tobacco included therein, is provided in U.S. Pat. Appl. Ser. Nos. 15/216,582 and 15/216,590, each filed July 21, 2016 and each to Davis et al.

The reservoir 312 may comprise a plurality of layers of nonwoven fibers formed into the shape of a tube encircling the interior of the outer body 314 of the cartridge 300. Thus, liquid components, for example, can be sorptively retained by the reservoir 312. The reservoir 312 is in fluid connection with the liquid transport element 324. Thus, the liquid transport element 324 may be configured to transport liquid from the reservoir 312 to the heating element 326 via capillary action or other liquid transport mechanism.

As illustrated, the liquid transport element 324 may be in direct contact with the heating element 326. As further illustrated in FIG. 3, the heating element 326 may comprise a wire defining a plurality of coils wound about the liquid transport element 324. In some embodiments the heating element 326 may be formed by winding the wire about the liquid transport element 324 as described in U.S. Pat. No. 9,210,738 to Ward et al. Further, in some embodiments the wire may define a variable coil spacing, as described in U.S. Pat. No. 9,277,770 to DePiano et al. Various embodiments of materials configured to produce heat when electrical current is applied therethrough may be employed to form the heating element 326. Example materials from which the wire coil may be formed include Kanthal (FeCrAl), Nichrome, Molybdenum disilicide (MoSi₂), molybdenum silicide (MoSi), Molybdenum disilicide doped with Aluminum (Mo(Si,Al)₂), graphite and graphite-based materials; and ceramic (e.g., a positive or negative temperature coefficient ceramic).

However, various other embodiments of methods may be employed to form the heating element 326, and various other embodiments of heating elements may be employed in the atomizer 310. For example, a stamped heating element may be employed in the atomizer, as described in U.S. Pat. App. Pub. No. 2014/0270729 to DePiano et al. Further to the above, additional representative heating elements and materials for use therein are described in U.S. Pat. No. 5,060,671 to Counts et al.; U.S. Pat. No. 5,093,894 to Deevi et al.; U.S. Pat. No. 5,224,498 to Deevi et al.; U.S. Pat. No. 5,228,460 to Sprinkel Jr., et al.; U.S. Pat. No. 5,322,075 to Deevi et al.; U.S. Pat. No. 5,353,813 to Deevi et al.; U.S. Pat. No. 5,468,936 to Deevi et al.; U.S. Pat. No. 5,498,850 to Das; U.S. Pat. No. 5,659,656 to Das; U.S. Pat. No. 5,498,855 to Deevi et al.; U.S. Pat. No. 5,530,225 to Hajaligol; U.S. Pat. No. 5,665,262 to Hajaligol; U.S. Pat. No. 5,573,692 to Das et al.; and U.S. Pat. No. 5,591,368 to Fleischhauer et al. Further, chemical heating may be employed in other embodiments. Various additional examples of heaters and materials employed to form heaters are described in U.S. Pat. No. 8,881,737 to Collett et al.

A variety of heater components may be used in the present aerosol delivery device. In various embodiments, one or more microheaters or like solid state heaters may be used. Microheaters and atomizers incorporating microheaters suitable for use in the presently disclosed devices are described in U.S. Pat. No. 8,881,737 to Collett et al.

The first heating terminal 320 and the second heating terminal 321 (e.g., negative and positive heating terminals) are configured to engage opposing ends of the heating element 326 and to form an electrical connection with the control body 200 (see, e.g., FIG. 2) when the cartridge 300 is connected thereto. Further, when the control body 200 is coupled to the cartridge 300, the electronic control component 306 may form an electrical connection with the control body through the control component terminal 304. The control body 200 may thus employ the electronic control component 212 (see, FIG. 2) to determine whether the cartridge 300 is genuine and/or perform other functions. Further, various examples of electronic control components and functions performed thereby are described in U.S. Pat. App. Pub. No. 2014/0096781 to Sears et al.

During use, a user may draw on the mouthpiece 316 of the cartridge 300 of the aerosol delivery device 100 (see, FIG. 1). This may pull air through an opening in the control body 200 (see, e.g., FIG. 2) or in the cartridge 300. For example, in one embodiment an opening may be defined between the coupler 202 and the outer body 204 of the control body 200 (see, e.g., FIG. 2), as described in U.S. Pat. No. 9,220,302 to DePiano et al. However, the flow of air may be received through other parts of the aerosol delivery device 100 in other embodiments. As noted above, in some embodiments the cartridge 300 may include the flow director 308. The flow director 308 may be configured to direct the flow of air received from the control body 200 to the heating element 326 of the atomizer 310.

A sensor in the aerosol delivery device 100 (e.g., the flow sensor 210 in the control body 200; see, FIG. 2) may sense the puff. When the puff is sensed, the control body 200 may direct current to the heating element 326 through a circuit including the first heating terminal 320 and the second heating terminal 321. Accordingly, the heating element 326 may vaporize the aerosol precursor composition directed to an aerosolization zone from the reservoir 312 by the liquid transport element 324. Thus, the mouthpiece 326 may allow passage of air and entrained vapor (i.e., the components of the aerosol precursor composition in an inhalable form) from the cartridge 300 to a consumer drawing thereon.

Various other details with respect to the components that may be included in the cartridge 300 are provided, for example, in U.S. Pat. App. Pub. No. 2014/0261495 to DePiano et al. Additional components that may be included in the cartridge 300 and details relating thereto are provided, for example, in U.S. Pat. Pub. No. 2015/0335071 to Brinkley et al., filed May 23, 2014.

Various components of an aerosol delivery device according to the present disclosure can be chosen from components described in the art and commercially available. Reference is made for example to the reservoir and heater system for controllable delivery of multiple aerosolizable materials in an electronic smoking article disclosed in U.S. Pat. App. Pub. No. 2014/0000638 to Sebastian et al.

In another embodiment substantially the entirety of the cartridge may be formed from one or more carbon materials, which may provide advantages in terms of biodegradability and absence of wires. In this regard, the heating element may comprise carbon foam, the reservoir may comprise carbonized fabric, and graphite may be employed to form an electrical connection with the power source and control component. An example embodiment of a carbon-based cartridge is provided in U.S. Pat. App. Pub. No. 2013/0255702 to Griffith et al.

However, in some embodiments it may be desirable to provide aerosol delivery devices with additional user control. For example, it may be desirable to allow a user to control the type or intensity of flavor of the vapor produced by the aerosol delivery device. Accordingly, embodiments of the present of the present disclosure include features configured to allow a user to customize the operation of aerosol delivery devices.

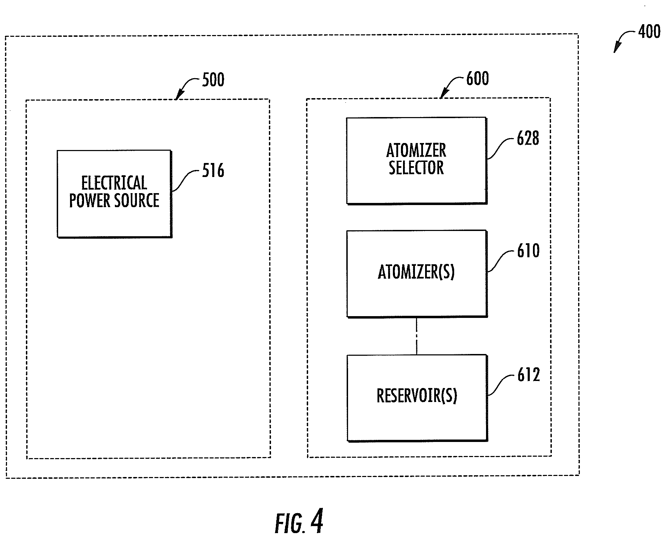

In this regard, FIG. 4 schematically illustrates an embodiment of an aerosol delivery device 400 according to an example embodiment of the present disclosure. As illustrated in FIG. 4, the aerosol delivery device 400 may include an electrical power source 516, one or more atomizers 610, and one or more reservoirs 612. The aerosol delivery device 400 may further include any of the other components described above. Additionally, the aerosol delivery device 400 may include an atomizer selector 628. As described hereinafter, the atomizer selector 628 may be configured to allow a user to customize a vapor produced by the aerosol delivery device 400.

As illustrated, in one embodiment the aerosol delivery device 400 may include a control body 500, which may include the electrical power source 516. Further, the aerosol delivery device 400 may include a cartridge 600, which may include the one or more atomizers 610, the one or more reservoirs 612, and the atomizer selector 628. However, in other embodiments the aerosol delivery device may not include a separate cartridge and control body or the components of the cartridge and the control body may be distributed in a differing manner. For example, the atomizer selector may be partially or fully included in the control body.

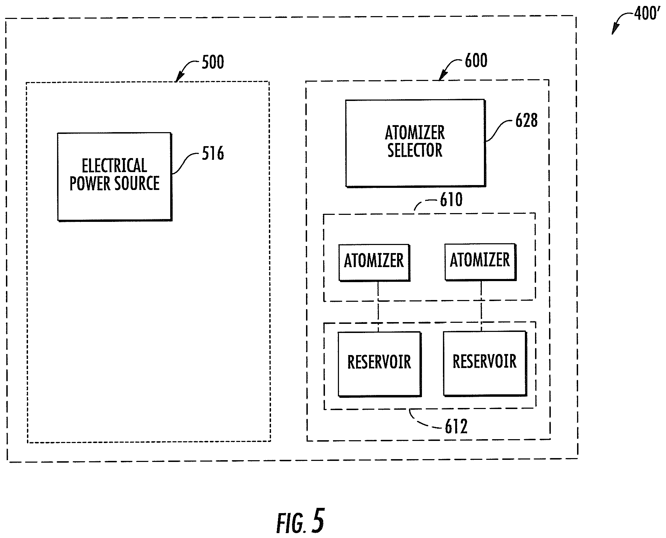

FIG. 5 illustrates an embodiment of the aerosol delivery device 400' including a plurality of the atomizers 610 and a plurality of the reservoirs 612. Example embodiments of aerosol delivery devices including multiple atomizers are described in U.S. Pat. Appl. Pub. No. 2014/0000638 to Sebastian et al. Each of the atomizers 610 may be in fluid communication with a respective one of the reservoirs 612. Further, the atomizer selector 628 may be configured to provide for selection of one or more of the atomizers 610 to which electrical current is directed to produce a vapor therefrom. In this regard, the electrical current may be provided by the electrical power source 516 and selectively directed to one or more of the atomizers 610 by the atomizer selector 628.

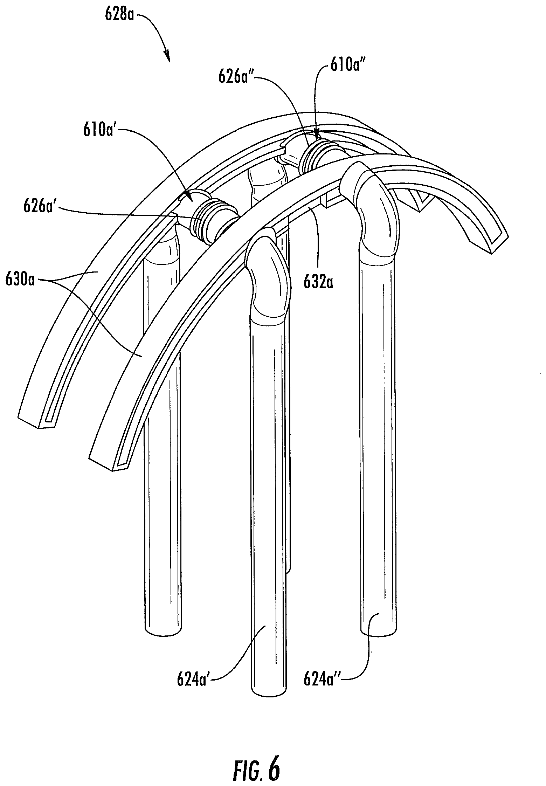

As may be understood, the atomizer selector 628 may selectively direct the electrical current to one or more of the atomizers 610 by any of a variety of mechanisms. In one embodiment selective direction of the electrical current to one or more of the atomizers 610 may be conducted mechanically. In this regard, FIG. 6 illustrates an embodiment of the atomizer selector 628a configured to mechanically select the one or more atomizers 610a to which the electrical current is directed. The atomizer selector 628a may include one or more guide tracks 630a, which may extend parallel to one another. First and second atomizers 610a', 610a" (collectively, "atomizers 610a") may be engaged with the guide tracks 630a. However, as may be understood, a greater number of atomizers may be engaged with the guide tracks 630a in other embodiments. Each of the atomizers 610a may include a liquid transport element 624a', 624a" and a heating element 626a', 626a" engaged therewith.

The atomizers 610a may be moveable relative to the guide tracks 630a. Each of the atomizers 610a may be moveable simultaneously, as a single unit, relative to the guide tracks 630a. In one embodiment the guide tracks 630a may move and the atomizers 610a may remain stationary. Alternatively, the guide tracks 630a may be stationary and the atomizers 610a may move. Regardless, relative motion between the guide tracks 630a and the atomizers 610a allows the atomizer selector 628a to selectively form an electrical connection with one or more of the atomizers. In this regard, the guide tracks 630a may respectively include one or more connection sections 632a. When one of the atomizers 610a engages the connection sections 632a, an electrical connection is formed therewith. Thereby, electrical current may be directed thereto. For example, the connection sections 632a may engage the ends of the heating elements of the atomizers 610a.

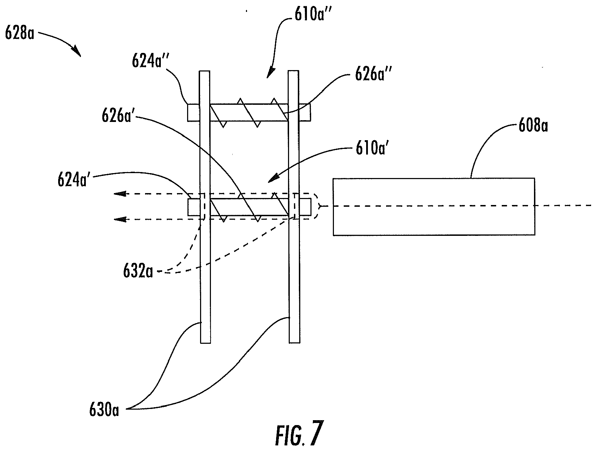

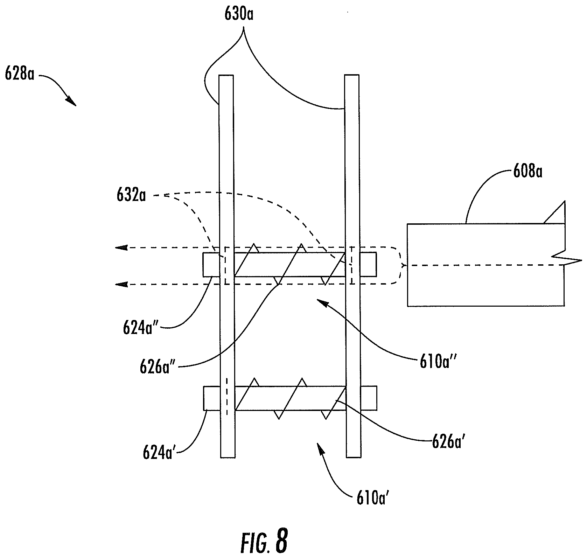

In this regard, FIG. 6 illustrates the atomizer selector 628a in an intermediate configuration wherein none of the atomizers 610a is engaged with the connection sections 632a. FIGS. 7 and 8 illustrate overhead views of the atomizer selector 628a in connected configurations. In particular, FIG. 7 illustrates the atomizer selector 628a and the atomizers 610a in a configuration wherein the first atomizer 610a' is engaged with the connection section 632a. In this configuration, the second atomizer 610a" is electrically disconnected. Thereby, electrical current may be directed to the first heating element 626a' to produce a vapor from the aerosol precursor composition retained in the one of the reservoirs 612a (see, FIG. 5) in fluid communication with the first liquid transport element 624a'. At this time electrical current may not be directed to the second heating element 626a", such that only the first heating element 626a' is activated.

Conversely, FIG. 8 illustrates the atomizer selector 628a and the atomizers 610a in a configuration wherein the second atomizer 610a" is engaged with the connection section 632a. In this configuration, the first atomizer 610a' is electrically disconnected. Thereby, electrical current may be directed to the second heating element 626a" to produce a vapor from the aerosol precursor composition retained in the one of the reservoirs 612a (see, FIG. 5) in fluid communication with the second liquid transport element 624a". At this time electrical current may not be directed to the first heating element 626a', such that only the second heating element 626a" is activated.

In some embodiments the atomizer selector 628a may be further configured to alter a position of the atomizers 610a with respect to an airflow path through the aerosol delivery device. In this regard, as further illustrated in FIGS. 7 and 8, the selected one of the atomizers 610a may be positioned in the airflow path through the aerosol delivery device. For example, as illustrated, in some embodiments the selected one of the atomizers 610a may be aligned with a flow director 608a by the atomizer selector 628a. Conversely, the one or more atomizers 610a that are not selected may be positioned outside of the airflow path through the aerosol delivery device or at a fringe thereof.

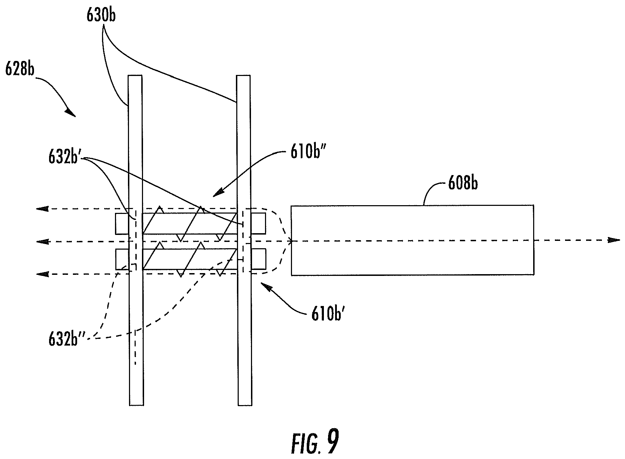

Note that in the embodiment described above, the atomizer selector 628a is configured to select one of the atomizers 610a, which is electrically connected and positioned in the airflow path. However, in other embodiments the atomizer selector may be configured to provide for selection of multiple atomizers 610a. For example, FIG. 9 illustrates an embodiment of the atomizer selector 628b wherein each guide track 630b includes multiple connection sections 632b', 632b". The atomizers 610b may be moveable relative to the guide tracks 630b such that multiple atomizers may be engaged with the connection sections at the same time. However, as may be understood, the atomizers 610b may be moved relative to the guide rails to select an individual atomizer.

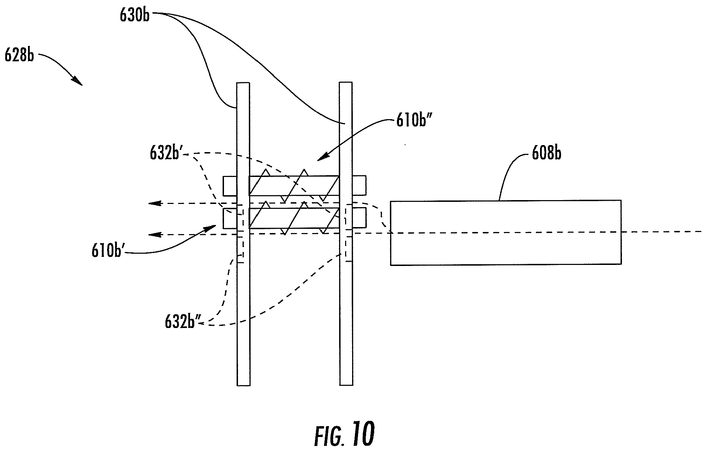

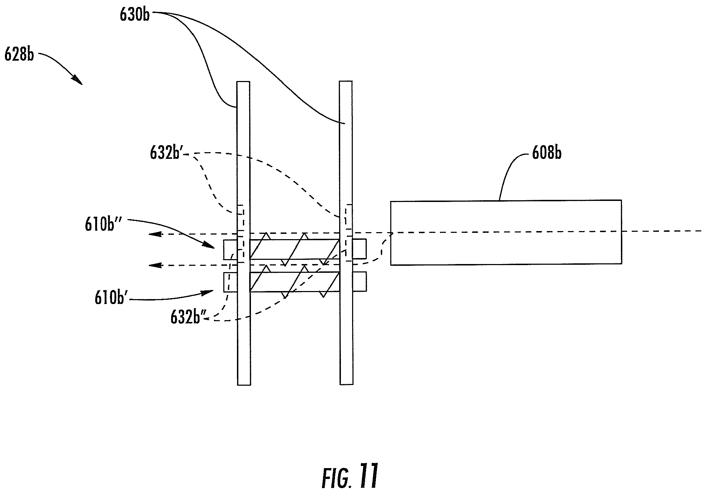

For example, FIG. 10 illustrates the atomizer selector 628b and the atomizers 610b in a configuration wherein the first atomizer 610b' is engaged with a first connection section 632b'. In this configuration, the second atomizer 610b" is electrically disconnected. Thereby, electrical current may be directed to the first atomizer 610b'. Conversely, FIG. 11 illustrates the atomizer selector 628b and the atomizers 610b in a configuration wherein the second atomizer 610b" is engaged with a second connection section 632b". In this configuration, the first atomizer 610b' is electrically disconnected. Thereby, electrical current may be directed to the second heating element 626b". Accordingly, one or more of the atomizers 610b may be selected for atomization. As further illustrated in FIGS. 9-11, the selected atomizers 610b may be aligned with the airflow path through the aerosol delivery device, whereas the atomizers that are not selected may be moved outside of the airflow path or to the fringe thereof.

Movement of the atomizers relative to the guide tracks in the embodiments described above may occur via a variety of actuators. For example a knob may be rotated to move the atomizers relative to the guide tracks. However, in other embodiments a slider, a switch, or any other actuator may move the atomizers relative to the guide tracks. Further, although guide tracks defining a curved configuration are illustrated, various other mechanisms may be employed in other embodiments with corresponding actuators such as switches, sliders, or any other actuator which may provide for any type of relative motion. Thus, for example, movements of the atomizer selector and/or atomizers may be linear or rotational and may involve axial pushing/pulling of the actuator and/or rotation thereof.

A user may be provided with feedback when the atomizers 610a, 610b engage the connection sections 632a, 632b. The feedback may be mechanical. For example, when one of the atomizers engages one of the connection sections, additional effort may be required to cause relative movement between the atomizers and the guide tracks. Additionally or alternatively, the feedback may be electrical. For example, the aerosol delivery device may include an indicator that indicates which atomizer(s) is/are engaged with the connection sections. In another embodiment the feedback may comprise haptic feedback as described, for example, in U.S. Pat. Appl. Pub. No. 2015/0020825 to Galloway et al.

As described above, in one embodiment the atomizer selector may selectively direct the electrical current to one or more of the atomizers via a mechanical apparatus that may move the atomizers relative to one or more guide tracks. However, in other embodiments the selective direction of the current to one more of the atomizers may be conducted electrically.

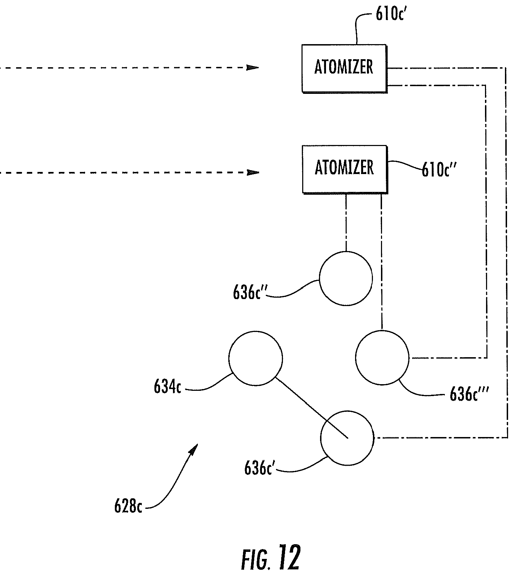

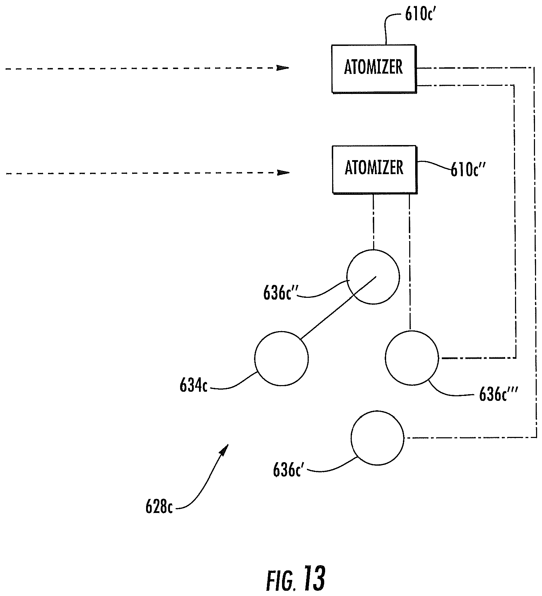

In this regard, FIG. 12 illustrates an atomizer selector 628c according to an additional example embodiment of the present disclosure. In this embodiment the atomizer selector 628c comprises a switch 634c. The atomizer selector 628c may further include a first contact 636c' and a second contact 636c". The first contact 636c' may be electrically connected to a first atomizer 610c'. The second contact 636c" may be electrically connected to a second atomizer 610c". Thereby, the switch 634c may be employed to provide for selection of which of the atomizers 610c', 610c" to which electrical current is directed. In this regard, as illustrated in FIG. 12, when the switch 634c engages the first contact 636c', electrical current may be directed to the first atomizer 610c'. Conversely, as illustrated in FIG. 13, when the switch 634c engages the second contact 636c", electrical current may be directed to the second atomizer 610c".

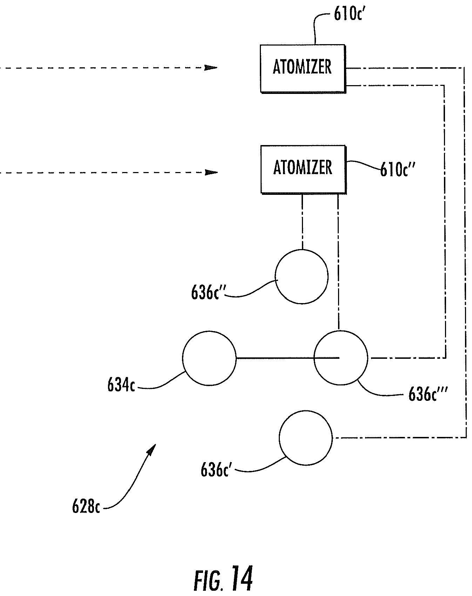

Further, in some embodiments the switch 634c may allow for simultaneous selection of multiple atomizers, in order to direct current to the multiple atomizers simultaneously when selected by the user. In this regard, the atomizer selector 628c may further comprise a third contact 636c‴. The third contact 636c‴ may be electrically connected to both the first atomizer 610c' and the second atomizer 610c". Thereby, as illustrated in FIG. 14, when the switch 634c engages the third contact 636c‴, electrical current may be directed to the first atomizer 610c' and the second atomizer 610c". Accordingly, the atomizer selector 628c may provide for selection of one or more of the atomizers 610c', 610c" to which electrical current is directed and which may be in fluid communication with a respective reservoir 612 (see, FIG. 5) to produce a vapor therefrom.

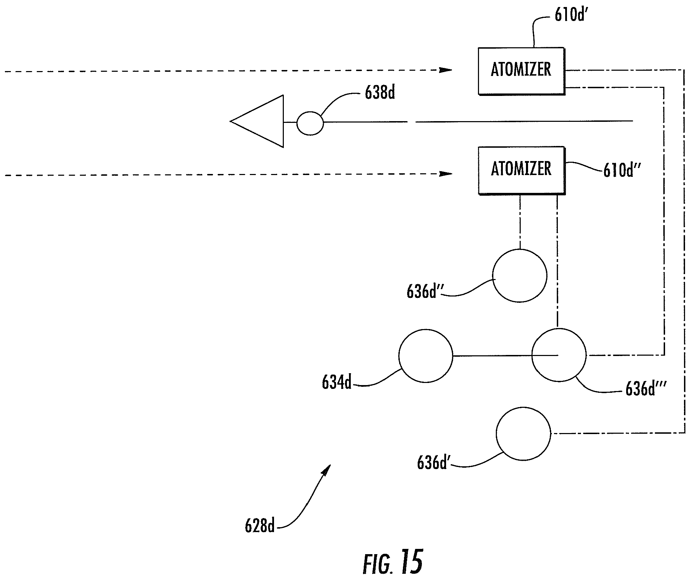

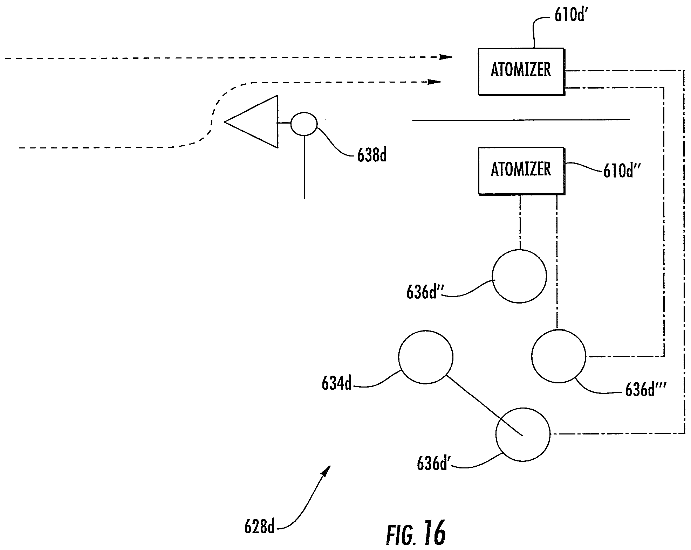

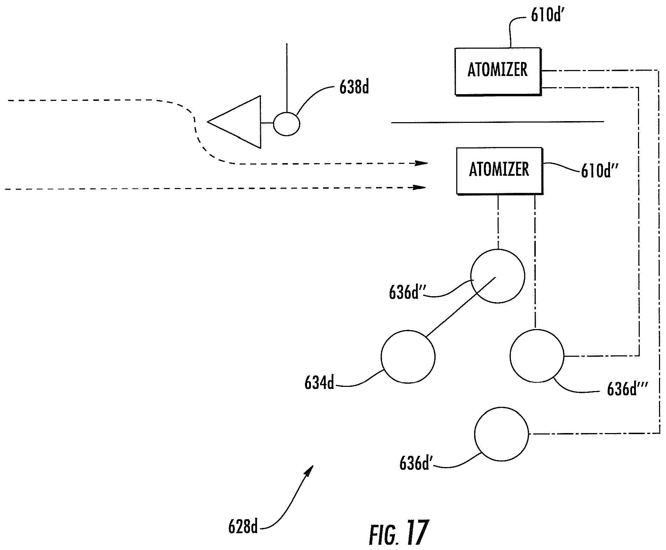

As illustrated in FIGS. 15-17, in some embodiments the atomizer selector 628d may further comprise a valve 638d configured to selectively direct the airflow path at one or more of the atomizers 610d', 610d". FIG. 15 illustrated the valve 638d in a neutral configuration, which may be employed when the switch 634d engages the third contact 636d"'. Thereby, the airflow may be directed around the valve 638d to each of the atomizers 610d', 610d", to which the electrical current is also directed. However, when the electrical current is directed to the first atomizer 610d', as illustrated in FIG. 16, the valve 638d may block flow to the second atomizer 610d" or otherwise directed airflow to the first atomizer 610d'. Conversely, when the electrical current is directed to the second atomizer 610d", as illustrated in FIG. 17, the valve 638d may block flow to the first atomizer 610d' or otherwise directed airflow to the second atomizer 610d".

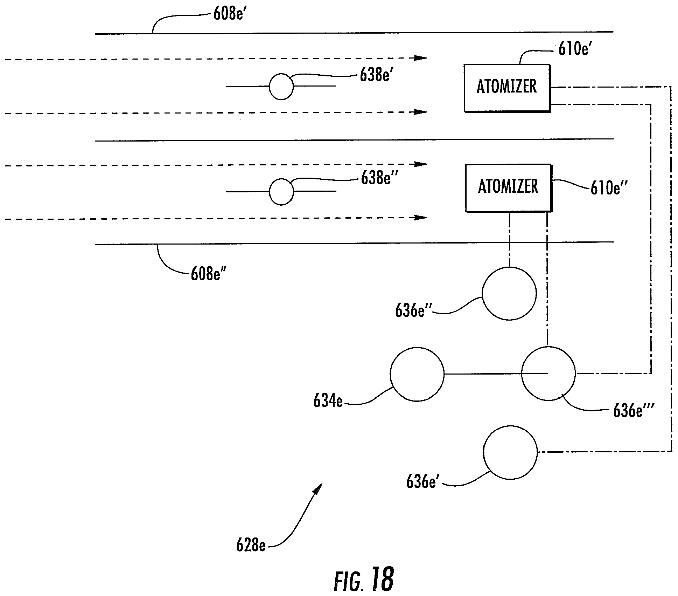

Additionally, although a single valve is described above as directing the airflow, in another embodiment the atomizer selector may include multiple valves. For example, as illustrated in the atomizer selector 628e of FIGS. 18-20, each atomizer 610e', 610e" may include a valve 638e', 638e" associated therewith. By way of further example, each atomizer 610', 610" may include a respective flow director 608e', 608e" configured to direct airflow thereto, and each flow director may include one of the valves 638e', 638e" associated therewith, such that airflow may be selectively directed to one or more of the atomizers.

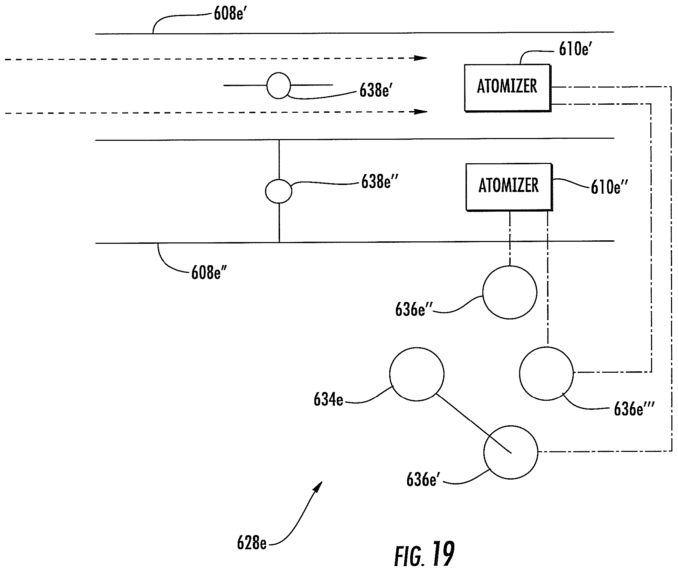

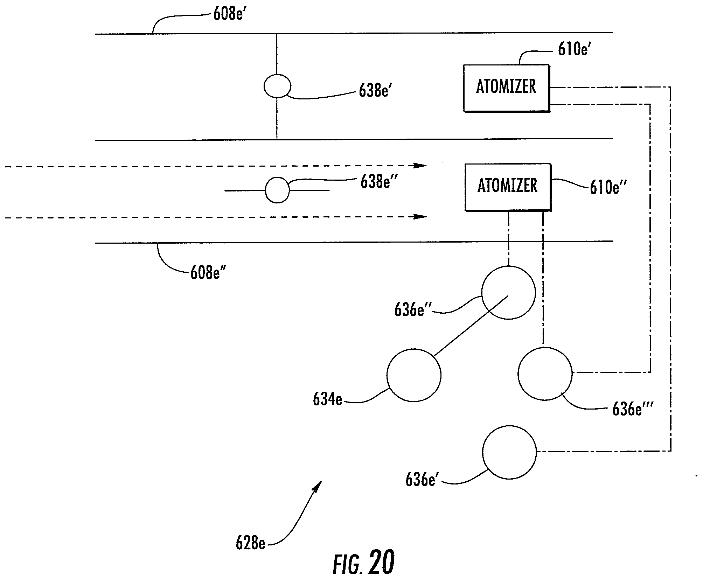

In this regard, FIG. 18 illustrates each of the valves 638e', 638e" in an open configuration such that airflow may be directed through the flow directors 608e', 608e" to each of the atomizers 610e', 610e". Further, the switch 634e is configured to direct electrical current to each of the atomizers 610e', 610e" via the third contact 636e‴, such that each of the atomizers may be activated. FIG. 19 illustrates the first valve 638e' in an open configuration and the second valve 638e" in a closed configuration such that airflow may be directed through the first flow director 608e' to the first atomizer 610e', but not through the second flow director 638e" to the second atomizer 610e". Further, the switch 634e is configured to direct electrical current to only the first atomizer 610e' via the first contact 636e'. Conversely, FIG. 20 illustrates the second valve 638e" in an open configuration and the first valve 638e' in a closed configuration such that airflow may be directed through the second flow director 608e" to the second atomizer 610e", but not through the first flow director 638e' to the first atomizer 610e'. Further, the switch 634e is configured to direct electrical current to only the second atomizer 610e' via the second contact 636e". Thus, the airflow and electrical current may be selectively directed to one or more of the atomizers 610e using the atomizer selector 628e comprising a plurality of valves 638e', 638e" and the switch 634e in some embodiments.

Note that although the switches are schematically illustrated as a manual switch, the switches may comprise a circuit or circuit board performing the same function. Thus, for example, the switches may perform the switching function fully electronically, without requiring a mechanical movement, in some embodiments. Further, although one or more valves are described as being employed to direct airflow to one or more selected atomizers, in other embodiments the position of the atomizers may be adjusted in order to move the atomizers into and out of the airflow in a manner corresponding to that described above.

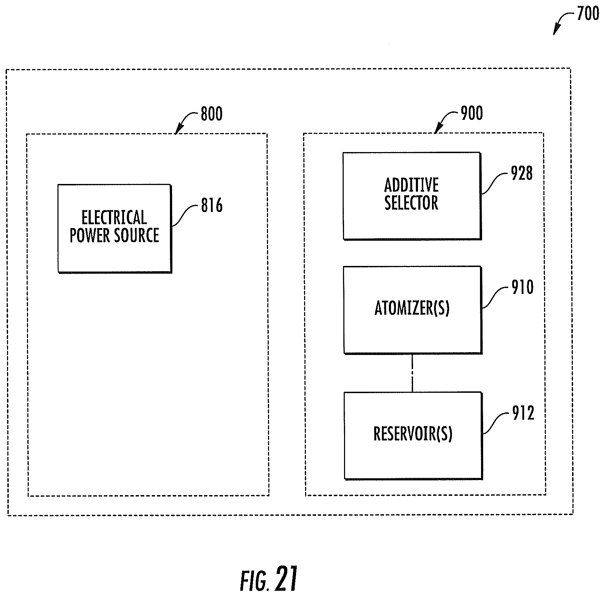

An aerosol delivery device 700 according to the invention is illustrated in FIG. 21. As schematically illustrated, the aerosol delivery device 700 may include an electrical power source 816, one or more atomizers 910, and one or more reservoirs 912. The aerosol delivery device 700 may further include any of the other components described above. Additionally, the aerosol delivery device 700 may include an additive selector 928. As described hereinafter, the additive selector 928 is configured to allow a user to customize a vapor produced by the aerosol delivery device 700 by providing for selection of one or more of a plurality of additives added to the vapor produced by the atomizer(s) 910 from the aerosol precursor composition retained in the reservoir(s) 912.

As illustrated, in one embodiment the aerosol delivery device 700 may include a control body 800, which may include the electrical power source 816. Further, the aerosol delivery device 700 may include a cartridge 900, which may include the one or more atomizers 910, the one or more reservoirs 912, and the additive selector 928. However, in other embodiments the aerosol delivery device may not include a separate cartridge and control body or the components of the cartridge and the control body may be distributed in a differing manner. For example, the additive selector may be partially or fully included in the control body.

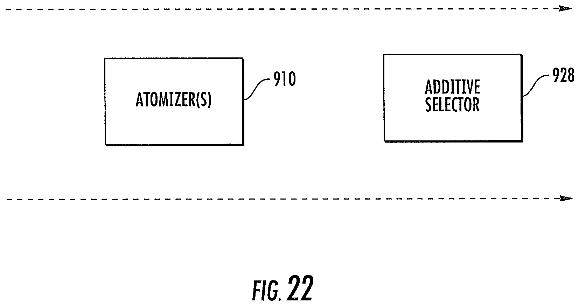

As illustrated in FIG. 22, in some embodiments the additive selector 928 may be positioned downstream of the atomizer(s) 910. Thereby, the one or more additives produced by the additive selector 928 are added to the vapor produced by the atomizer 910. However, in another embodiment the additive selector may be positioned upstream of the atomizer or at substantially the same location along the airflow path. Thus, the vapor and the additive selected may combine regardless of the relative positions of the atomizer(s) 910 and the additive selector 928. However, positioning the additive selector 928 downstream of the atomizer(s) 910 may be employed to release the additive from the additive selector 928 due, for example, to the heat and/or moisture provided by the vapor thereto.

In some embodiments the vapor produced by the atomizer(s) 910 may be flavorless and/or lack an active ingredient. In this embodiment, the additive selector 928 may add a flavor and/or active ingredient (e.g., a medication or nicotine) thereto. Thereby, a universal aerosol precursor composition may be used with any of a variety of additives.

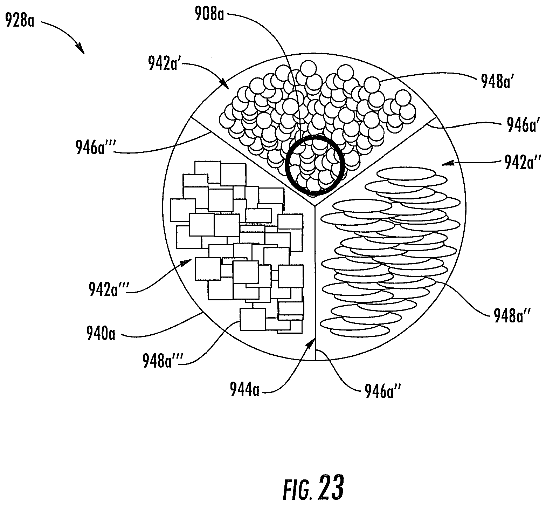

FIG. 23 illustrates an embodiment of the additive selector 928a, wherein the additive selector comprises a bed of solids 940a. As noted above, the additive selector 928a, and hence the bed of solids 940a, may be positioned downstream of the at least one atomizer 910 (see, FIG. 22) in terms of an airflow path through the aerosol delivery device 700 (see, FIG. 21). The bed of solids 940 may include a plurality of compartments 942a', 942a", 942a‴ (collectively and generically, "compartments 942a"). In this regard, a divider 944a may include one or more partitions 946a', 946a", 946a‴ (collectively and generically, "partitions 946a") that separate the compartments 942a. In the illustrated embodiment the divider 944a includes three partitions 946a that divide the additive selector 928a into three compartments 942a. However, in other embodiments a greater or lesser number of partitions and corresponding compartments may be employed.

Each of the compartments 942a may include one or more solids 948a', 948a", 948a‴ (collectively and generically, "solids 948a"). In some embodiments the solids 948a received in one of the compartments 942a may differ from the solids received in each of the other compartments. Various embodiments of the solids 948a may be employed in the bed of solids 940a. In one embodiment the solids 948a may comprise a plurality of flavor-laden plastic solids. The solids may be provided in various forms including, by way of example, beads, pellets, shreds, or a porous monolith.

The aerosol delivery device 700, and in particular the cartridge 900 (see, FIG. 21), may further include a flow director. As illustrated in FIG. 23, the flow director 908a may direct airflow toward the additive selector 928a. Thus, in terms of the perspective illustrated in FIG. 23, airflow is directed into the page.

The additive selector 928a may be configured to selectively align the flow director 908a with one or more of the compartments 942a. For example, in FIG. 23 the flow director 908a is aligned with the first compartment 942a'. However, by rotating or otherwise moving the additive selector 928a relative to the flow director 908a, the flow director may be selectively aligned with any one of the other compartments 942a. Thereby, the additive selector 928a may add a selected one of a plurality of additives to the vapor produced by the atomizer(s) 910 (see, e.g., FIG. 22). In some embodiments an actuator (e.g., an electric motor) may move the additive selector relative to the flow director, whereas in other embodiments the additive selector may be manually moveable relative to the flow director.

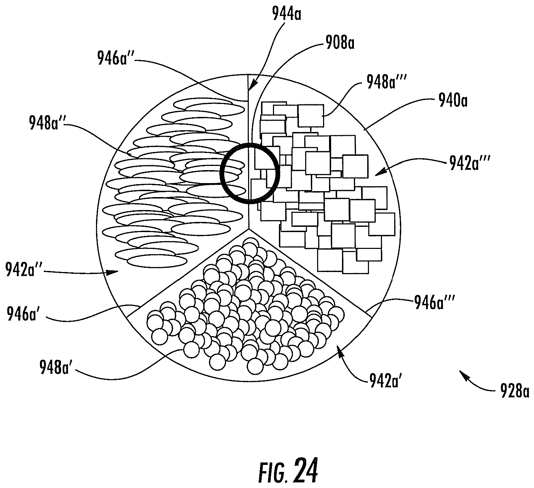

Further, in some embodiments the flow director 908a may be selectively aligned with multiple compartments 942a. For example, FIG. 24 illustrates the flow director 908a selectively aligned with a portion of the second compartment 942a" and a portion of the third compartment 942a"'. Accordingly, multiple additives may be added to the vapor at the same time in some positions of the flow director 908a relative to the additive selector 928a.

As noted above, in some embodiments the flow director 908a may be aligned with one or more of the compartments 942a of the additive selector 928a. Thereby, one or more additives may be added to the vapor directed through the one or more selected compartments 942a by the one or more corresponding solids 948a.

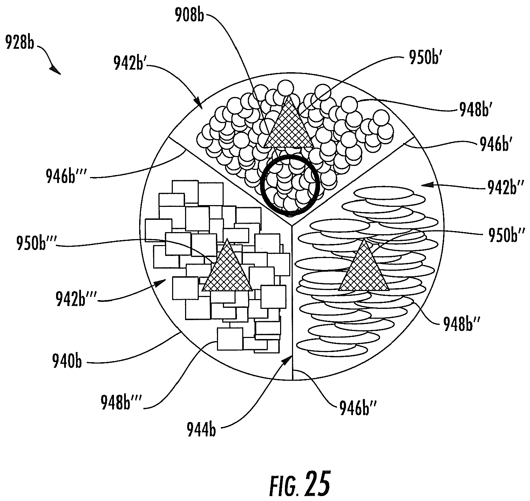

FIG. 25 illustrates an additional embodiment of the additive selector 928b. As illustrated, the additive selector 928b includes one or more additive heating elements 950b', 950b", 950b‴ (collectively and generically, "heating elements 950b"). The additive heating elements 950b are configured to selectively heat one or more portions of the bed of solids 940b. In this regard, the first additive heating element 950b' may be configured to heat the solids 948b' received in the first compartment 942b', the second additive heating element 950b" may be configured to heat the solids 948b" received in the second compartment 942b", and the third additive heating element 950b‴ may be configured to heat the solids 948b‴ received in the third compartment 942b'''. Accordingly, one or more of the additive heating elements 950b may be selectively activated to heat the solids 948b in the one or more corresponding compartments 942b. Thereby, heat produced by one or more of the additive heating elements 942b may release one or more additives from the bed of solids 940b that is added to the vapor produced by the atomizer(s) 910 (see, e.g., FIG. 22).

In this embodiment the additive selector 928b may be stationary relative to the atomizers 910 (see, e.g., FIG. 22). In this regard, one or more of the heating elements 950b may produce the additive, such that selective direction of the airflow through one or more portions of the bed of solids 940b may not be required. However, in some embodiments the additive selector 928b may be moveable relative to the atomizer(s) 910. Thereby, the airflow may be directed to the one or more compartment(s) 942b at which a heating element 950b is activated. Thus, for example, the flow director 908b may be included in the aerosol delivery device 700 (see, FIG. 21) to direct airflow to the one or more compartment(s) 942b at which a heating element 950b is activated.

In the embodiment illustrated in FIG. 25, airflow occurs into the page in terms of the illustrated orientation. In this regard, each of the compartments 942b is positioned beside one another, at the same point along the longitudinal length of the aerosol delivery device. Thus, the compartments 942b may be laterally distributed across the width of the aerosol delivery device.

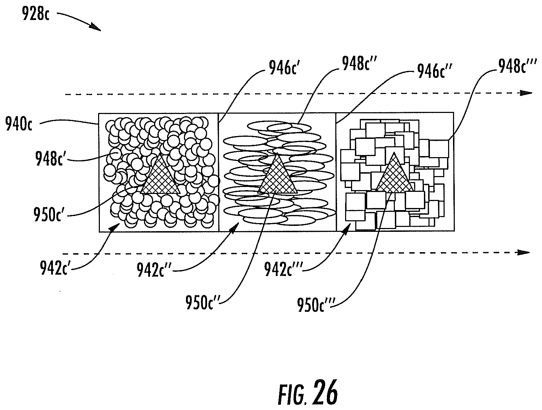

However, the compartments may be positioned relative to one another in other manners. For example, FIG. 26 illustrates an embodiment of the additive selector 928c including a bed of solids 940c. As noted above, the additive selector 928c, and hence the bed of solids 940c, may be positioned downstream of the at least one atomizer 910 (see, FIG. 22) in terms of an airflow path through the aerosol delivery device 700 (see, FIG. 21). The bed of solids 940c may include a plurality of compartments 942c', 942c", 942c‴ (collectively and generically, "compartments 942c"). In this regard, one or more partitions 946c', 946c" (collectively and generically, "partitions 946c") may separate the compartments 942c. In the illustrated embodiment two partitions 946c divide the bed of solids 940c into three compartments 942c. However, in other embodiments a greater or lesser number of partitions and corresponding compartments may be employed.

Each of the compartments 942c may include one or more solids 948c', 948c", 948c‴ (collectively and generically, "solids 948c"). In some embodiments the solids 948c received in one of the compartments 942b may differ from the solids received in each of the other compartments. Various embodiments of the solids 948c may be employed in the bed of solids. In one embodiment the solids 948c may comprise a plurality of flavor-laden plastic solids. The solids may be provided in various forms including, by way of example, beads, pellets, shreds, or a porous monolith.

The additive selector 928c includes one or more additive heating elements 950c', 950c", 950c‴ (collectively and generically, "heating elements 950c"). The additive heating elements 950c are configured to selectively heat one or more portions of the bed of solids 940c. In this regard, the first additive heating element 950c' may be configured to heat the solids 948c' received in the first compartment 942c', the second additive heating element 950c" may be configured to heat the solids 948c" received in the second compartment 942c", and the third additive heating element 950c‴ may be configured to heat the solids 948c‴ received in the third compartment 942c"'. Accordingly, one or more of the additive heating elements 950c may be selectively activated to heat the solids 948c in the one or more corresponding compartments 942c. Thereby, heat produced by one or more of the additive heating elements 942c may release one or more additives from the bed of solids 940c that is added to the vapor produced by the atomizer(s) 910 (see, e.g., FIG. 22).

Thus, the additive selector 928c may be substantially similar to the additive selector 928b of FIG. 25. However, as noted above, the additive selector 928b of FIG. 25 is configured with the compartments 942b distributed laterally across the width of the aerosol delivery device. Thus, the additive selector 928c of FIG. 26 differs in that the compartments 942c are distributed longitudinally along the longitudinal length of the aerosol delivery device. In this regard, airflow may be directed through the first compartment 942c' into the second compartment 942c", and then into and out of the third compartment 942c'". Thus, the compartments of the bed of solids including heating elements that release additives may be distributed in any of various manners in view of the additive being released into the vapor when one or more of the heaters are activated. In this regard, release of the additive does not require selective airflow therethrough.

As described above, in some embodiments of the present disclosure an aerosol delivery device may include an atomizer configured to produce a vapor from an aerosol precursor composition retained in a reservoir and an additive selector including a bed of solids configured to add an additive to the vapor. Various other details with respect to hybrid aerosol delivery devices, including embodiments of solids that may be included in the bed of solids are provided in U.S. Pat. App. Pub. Nos. 2015/0335070 and 2016/0073695 to Sears et al.

As noted above, embodiments of the present invention provide an additive selector including one or more additive heating elements that heat solids to produce one or more additives. Although the solids are described above as generally being received in a bed of solids, in other embodiments the configuration may vary. For example, as noted above, flavor-laden plastic solids may be heated to release an additive. In some embodiments one or more components of the aerosol delivery device (e.g., the mouthpiece and/or the flow director) may comprise flavor-laden plastic. Thereby, one or more additive heating elements and/or heat from the atomizer may be employed to heat the one or more components to release the additive. Further, one or more valves may be employed to selectively direct air to the components that are heated and produce an additive. In some embodiments such components including flavor-laden plastic may be replaceable or removable in order to allow a user to replenish or change the additive.

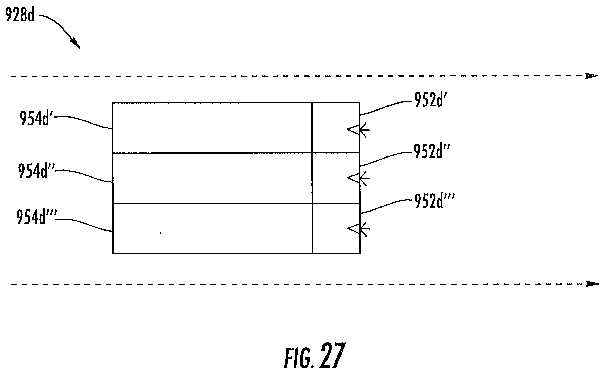

FIG. 27 illustrates an additional embodiment of the additive selector 928d. As illustrated, the additive selector 928d may include one or more bubble jet heads 952d', 952d", 952d‴ (collectively and generically, "bubble jet heads 952d"). The bubble jet heads 952d may be configured to provide for selection of one or more of a plurality of additives added to the vapor produced by the atomizer(s) 910 (see, e.g., FIG. 22).

Each of the bubble jet heads 952d may be in fluid communication with a respective additive reservoir 954d', 954d", 954d‴ (collectively and generically, "additive reservoirs 954d"). The additive reservoirs 954d may each contain an additive fluid (e.g., a flavor and/or active ingredient) which may be dispensed by the bubble jet heads 952d into the vapor produced by the atomizer(s) 910 (see, e.g., FIG. 22). In some embodiments the additive fluids retained in each of the additive reservoirs 954d may differ from one another. Accordingly, one or more of the bubble jet heads 952d may be selectively activated to add one or more additives to the vapor.

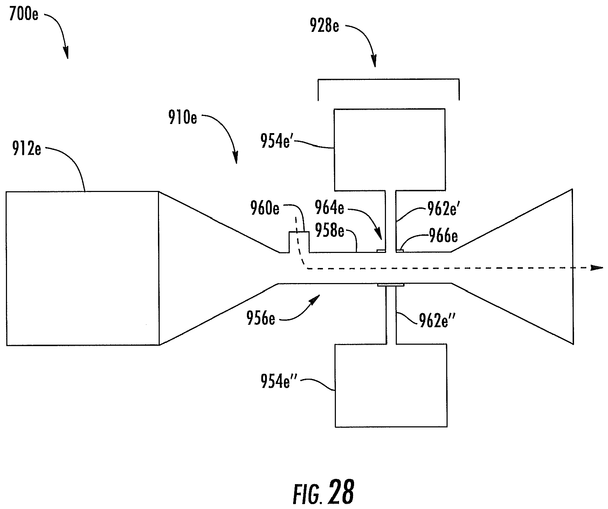

FIG. 28 illustrates an additional embodiment of the aerosol delivery device 700e. As illustrated, the atomizer 910e may comprise a Venturi nozzle 956e. When a user draws on the aerosol delivery device 700e, aerosol precursor composition may be drawn from the reservoir 912e through the Venturi nozzle 956e. The Venturi nozzle 956e may include a restriction 958e that accelerates the flow of the aerosol precursor composition therethrough and produces an area of low pressure. The low pressure may draw in outside air through an air inlet 960e that mixes with the aerosol precursor composition to produce a vapor.

Further, the additive selector 928e may be configured to provide for selection of one or more of a plurality of additives added to the vapor. In this regard, the additive selector 928e may comprise a plurality of channels 962e', 962e" (collectively and generically, "channels 962e") each configured to be in fluid communication with one of a plurality of additive reservoirs 954e', 954e" (collectively, "additive reservoirs 954e") and selectively configurable to be in fluid communication with the Venturi nozzle 956e.

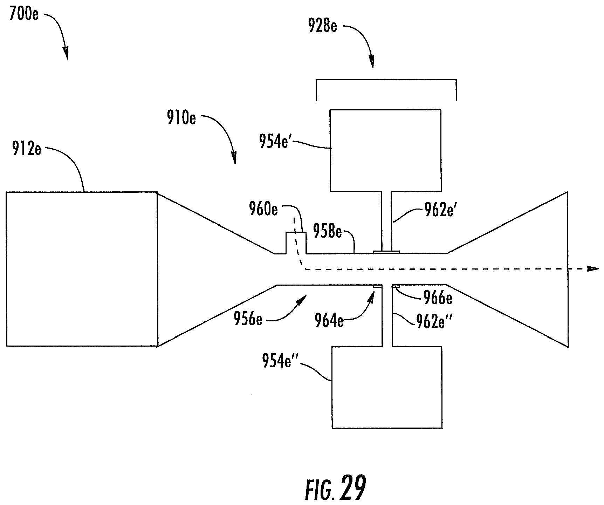

In this regard, the additive selector 910e may further comprise an additive valve 964e. The additive valve 964e may include at least one opening 966e configured to be selectively aligned with one or more of the channels 962e. For example, in FIG. 28 the first channel 962e' is in fluid communication with the Venturi nozzle 956e via the opening 966e in the additive valve 964e, whereas the second channel 962e" is out of fluid communication with the Venturi nozzle. Conversely, in FIG. 29 the second channel 962e" is in fluid communication with the Venturi nozzle 956e via the opening 966e in the additive valve 964e, whereas the first channel 962e' is out of fluid communication with the Venturi nozzle.

Thus, when one or more of the channels 962e is in fluid communication with the Venturi nozzle 956e and the user draws on the aerosol delivery device 700e, the low pressure at the restriction 958e may draw a fluid additive from each additive reservoir 954e in fluid communication therewith into the Venturi nozzle. Thereby, the one or more fluid additives may be vaporized and added to the vapor produced from the aerosol precursor composition received from the reservoir 912e. Note that the restriction 958e and/or the channels 962e may comprise capillary tubes configured to resist flow therethough except when a user draws on the aerosol delivery device 700e in order to prevent leakage therefrom.

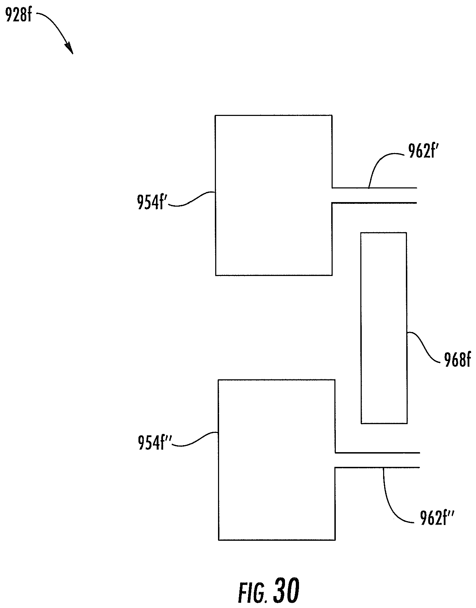

FIG. 30 illustrates an additional embodiment of the additive selector 928f. As illustrated, the additive selector 928f may include at least one additive reservoir 954f', 954f" (collectively and generically, "additive reservoirs 954f"). The additive reservoirs 954f may each contain an additive fluid (e.g., a flavor and/or active ingredient). In some embodiments the additive fluids retained in each of the additive reservoirs 954f may differ from one another.

Each additive reservoir 954f may be in fluid communication with a respective channel 962f', 962f" (collectively and generically, "channels 962f"). In the illustrated embodiment the additive selector 928f includes two additive reservoirs 954f and two corresponding channels 962f. However, a greater or lesser number of additive reservoirs and channels may be employed in other embodiments.

As illustrated in FIG. 30, the additive selector 928f may further include at least one crystal oscillator 968f. The crystal oscillator 968f may be configured to ultrasonically vibrate in contact with one or more of the channels 962f and/or the additive fluid received therein. For example, in one embodiment the crystal oscillator 968f may comprise a vibratory screen in fluid communication with one or more of the channels 962f. Thereby, the additive fluid in the one or more channels 962f in contact with the crystal oscillator 968f may be atomized to produce one or more additives added to the vapor produced by the atomizer(s) 910 (see, e.g., FIG. 22). In one embodiment a separate crystal oscillator may be associated with each of the channels, such that the crystal oscillators may be selectively activated to produce one or more additives. In another embodiment a single crystal oscillator may be employed to selectively atomize the additive fluid in one or more of multiple channels.

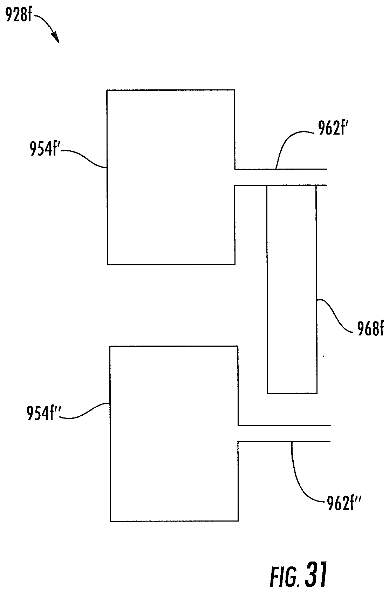

In this regard, the crystal oscillator 968f may be moveable relative to the channels 962f. For example, FIG. 31 illustrates the crystal oscillator 968f in contact with the first channel 962f' in order to atomize the fluid additive retained in the first additive reservoir 954f'. However, as may be understood, the crystal oscillator 968f may be moved relative to the second channel 962f" so as to additionally or alternatively atomize the fluid additive retained in the second additive reservoir 954f".

The embodiments of the additive selectors 928d-f described above with respect to FIGS. 27-31 may produce additives having a relatively large droplet size. Usage of relatively large droplets or particles may desirable in embodiments wherein the additive is configured to provide a flavor. In this regard, relatively larger droplets and particles may be easier for flavor receptors to recognize, and thus may provide a stronger flavor.

Embodiments of the aerosol delivery devices described above include an additive selector. In some embodiments the additive selector or a portion thereof (e.g., the bed of solids) may be detachable, refillable, and/or replaceable. Thereby, the additive may be replenished or exchanged for a differing type thereof. In some embodiments the additive may be configured for a single use (e.g., a single puff), which may be useful when the additive comprises a medication in order to provide the correct dosage thereof without risk of an overdose.

Embodiments of aerosol delivery devices described above include multiple atomizers. In some embodiments two or more of the atomizers may be operated simultaneously. In these embodiments the volume of vapor produced by the atomizers may be adjusted. For example, the amount of electrical current directed to each of the atomizers may be separately adjustable by the user. Further, in some embodiments of the aerosol delivery devices described above including multiple atomizers, the atomizers or components thereof (e.g., a liquid transport element and/or heating element) may be differently sized such that two or more atomizers define differing sizes and/or other properties.

In the embodiments described above, various atomizer selectors are employed to select atomizers, additives, valves, etc., in order to customize the operation of aerosol delivery devices. Such selections may be conducted mechanically, electrically, or via a combination thereof. In some embodiments an application may be provided that allows a mobile phone or other electronic device to communicate with the aerosol delivery device and make such selections via the application. Thereby, the atomizer selectors may be controlled via the application in some embodiments.

As described above, various embodiments of additives may be employed. By way of example, the additives may comprise flavors or active ingredients such as pharmaceuticals (e.g., albuterol or nicotine). Example flavors may include vanillin, ethyl vanillin, cream, tea, coffee, fruit (e.g., banana, berry, apple, cherry, strawberry, peach and citrus flavors, including lime and lemon), maple, menthol, chocolate, mint, peppermint, spearmint, wintergreen, nutmeg, clove, lavender, cardamom, ginger, honey, anise, sage, cinnamon, sandalwood, jasmine, cascarilla, cocoa, licorice, and flavorings of the type and character traditionally used for the flavoring of cigarette, cigar and pipe tobaccos.

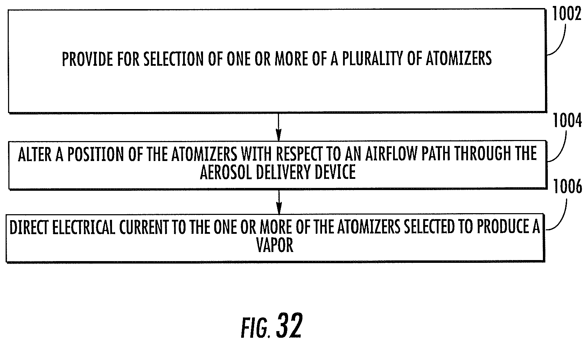

In an additional aspect a method for vapor production with an aerosol delivery device is provided. As illustrated in FIG. 32, the method may include providing for selection of one or more of a plurality of atomizers at operation 1002. Further, the method may include altering a position of the atomizers with respect to an airflow path through the aerosol delivery device at operation 1004. The method may additionally include directing electrical current to the one or more of the atomizers selected to produce a vapor at operation 1006.

In some embodiments altering a position of the atomizers with respect to the airflow path through the aerosol delivery device at operation 1004 may include selectively directing the airflow path at the one or more of the atomizers selected with the valve. Further, directing electrical current to the one or more of the atomizers selected to produce the vapor at operation 1006 comprises selectively forming an electrical connection with the one or more of the atomizers selected. The method may further include providing for selection of one or more of a plurality of additives and adding the one or more additives selected to the vapor.

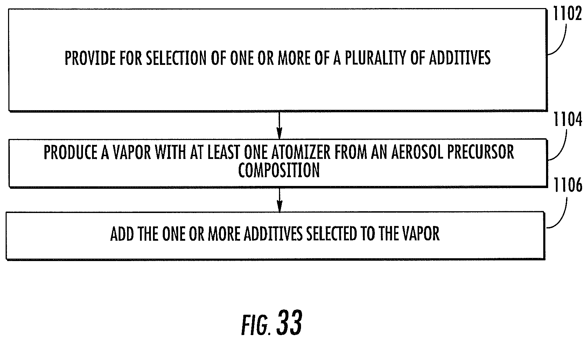

According to the invention, a method for vapor production with an aerosol delivery device is provided. As illustrated in FIG. 33, the method may include providing for selection of one or more of a plurality of additives at operation 1102. Further, the method may include producing a vapor with at least one atomizer from an aerosol precursor composition at operation 1104. The method includes adding the one or more additives selected to the vapor.

According to the invention, the method comprises forming the one or more additives from a bed of solids. Providing for selection of one or more of the additives at operation 1102 may include providing for selective alignment of a Venturi nozzle with one or more channels respectively in fluid communication with one of a plurality of additive reservoirs. Further, adding the one or more additives selected to the vapor at operation 1106 may include activating at least one crystal oscillator. Producing the vapor with at least one atomizer at operation 1104 may include providing for selection of one or more of a plurality of atomizers. The method may further include altering a position of the atomizers with respect to an airflow path through the aerosol delivery device and directing electrical current to the one or more of the atomizers selected to produce a vapor.

## Claims

1. An aerosol delivery device (700), comprising:
at least one atomizer (910) configured to produce a vapor from an aerosol precursor composition; and
an additive selector (928) configured to provide for selection of one or more of a plurality of additives added to the vapor,
wherein the additive selector (928) comprises:
a bed of solids (940), and
one or more additive heating elements (950c) configured to selectively heat one or more portions of the bed of solids (940).

2. The aerosol delivery device of Claim 1, wherein the bed of solids (940) is positioned downstream of the at least one atomizer (910) in terms of an airflow path, and optionally wherein the bed of solids (940) comprises a plurality of flavor-laden plastic solids, and optionally wherein the bed of solids (940) comprises a plurality of compartments (942c) separated by one or more partitions (946c), and optionally further comprising a flow director (908b), the additive selector (928) being configured to selectively align the flow director (908b) with one or more of the compartments (942c).

3. The aerosol delivery device of Claim 1 or 2, wherein the at least one atomizer (910) comprises a Venturi nozzle (956e), and
wherein the additive selector (928) comprises a plurality of channels (926e) each configured to be in fluid communication with one of a plurality of additive reservoirs (954e) and selectively configurable to be in fluid communication with the Venturi nozzle (956e).

4. The aerosol delivery device of any one of Claims 1 to 3, wherein the additive selector (928) comprises at least one crystal oscillator (968f).

5. The aerosol delivery device of any one of Claims 1 to 4, wherein the at least one atomizer (910) includes a plurality of atomizers (910) each configured to be in fluid communication with a respective one of a plurality of reservoirs (954e), and optionally further comprising an atomizer selector (628) configured to:
provide for selection of one or more of the atomizers (910) to which electrical current is directed to produce the vapor therefrom; and
alter a position of the atomizers (910) with respect to an airflow path through the aerosol delivery device (700).

6. A method for vapor production with an aerosol delivery device (700), the method comprising:
forming one or more of a plurality of additives from a bed of solids (940);
providing for selection of the one or more of a plurality of additives;
selectively heating one or more portions of the bed of solids (940) to release the selected one or more of the plurality of additives from the bed of solids (940);
producing a vapor with at least one atomizer (910) from an aerosol precursor composition; and
adding the released one or more of the plurality of additives selected to the vapor.

7. The method of Claim 6, wherein providing for selection of one or more of the additives comprises providing for selective alignment of a Venturi nozzle (956e) with one or more channels (926e) respectively in fluid communication with one of a plurality of additive reservoirs (954e).

8. The method of Claim 6 or 7, wherein adding the one or more additives selected to the vapor comprises activating at least one crystal oscillator (968f), and optionally wherein producing the vapor with at least one atomizer (910) comprises providing for selection of one or more of a plurality of atomizers (910), and optionally further comprising altering a position of the atomizers (910) with respect to an airflow path through the aerosol delivery device (700); and
directing electrical current to the one or more of the atomizers (910) selected to produce a vapor.

## Patentansprüche

1. Eine Aerosolabgabevorrichtung (700), umfassend:
mindestens einen Zerstäuber (910), welcher dazu ausgebildet ist, einen Dampf aus einer Aerosol-Precursor-Zusammensetzung zu erzeugen; und
einen Additivselektor (928), welcher dazu ausgebildet ist, eine Selektion eines oder mehrerer einer Mehrzahl von Additiven bereitzustellen, welche dem Dampf hinzugefügt werden,
wobei der Additivselektor (928) umfasst:
ein Feststoffbett (940) und
ein oder mehrere Additiv-Heizelemente (950c), welche dazu ausgebildet sind, einen oder mehrere Bereiche des Feststoffbetts (940) selektiv zu erwärmen.

2. Die Aerosolabgabevorrichtung nach Anspruch 1, wobei das Feststoffbett (940) bezogen auf einen Luftströmungspfad stromab des mindestens einen Zerstäubers (910) positioniert ist, und wobei optional das Feststoffbett (940) eine Mehrzahl von Flavour-beladenen Kunststofffeststoffen umfasst, und wobei optional das Feststoffbett (940) eine Mehrzahl von Abteilen (942c) umfasst, welche mittels einer oder mehrerer Trennwände (946c) voneinander getrennt sind, und optional ferner umfassend eine Strömungsleiteinrichtung (908b), wobei der Additivselektor (928) dazu ausgebildet ist, die Strömungsleiteinrichtung (908b) zu einem oder mehreren der Abteile (942c) auszurichten.

3. Die Aerosolabgabevorrichtung nach Anspruch 1 oder 2, wobei der mindestens eine Zerstäuber (910) eine Venturidüse (956e) umfasst, und wobei der Additivselektor (928) eine Mehrzahl von Kanälen (926e) umfasst, welche jeweils dazu ausgebildet sind, mit einem einer Mehrzahl von Additivreservoirs (954e) in Fluidverbindung zu stehen, und selektiv konfigurierbar sind, um mit der Venturidüse (956e) in Fluidverbindung zu stehen.

4. Die Aerosolabgabevorrichtung nach einem der Ansprüche 1 bis 3, wobei der Additivselektor (928) mindestens einen Kristalloszillator (968f) umfasst.

5. Die Aerosolabgabevorrichtung nach einem der Ansprüche 1 bis 4, wobei der mindestens eine Zerstäuber (910) eine Mehrzahl von Zerstäubern (910) umfasst, welche jeweils dazu ausgebildet sind, mit jeweils einem einer Mehrzahl von Reservoirs (954e) in Fluidverbindung zu stehen, und optional ferner umfassend einen Zerstäuberselektor (628), welcher zu Folgendem ausgebildet ist:
Bereitstellen einer Selektion eines oder mehrerer der Zerstäuber (910), denen ein elektrischer Strom zugeleitet wird, um aus denselben den Dampf zu erzeugen; und
Ändern einer Position der Zerstäuber (910) in Bezug auf einen Luftströmungspfad durch die Aerosolabgabevorrichtung (700) hindurch.

6. Ein Verfahren zur Dampferzeugung mit einer Aerosolabgabevorrichtung (700), wobei das Verfahren umfasst:
Bilden eines oder mehrerer einer Mehrzahl von Additiven aus einem Feststoffbett (940);
Bereitstellen einer Selektion des einen oder der mehreren einer Mehrzahl von Additiven;
selektives Erwärmen eines oder mehrerer Bereiche des Feststoffbetts (940), um das selektierte eine oder die selektierten mehreren der Mehrzahl von Additiven aus dem Feststoffbett (940) freizusetzen;
Erzeugen eines Dampfes mit mindestens einem Zerstäuber (910) aus einer Aerosol-Precursor-Zusammensetzung; und
Hinzufügen des freigesetzten einen oder der freigesetzten mehreren der Mehrzahl von Additiven, welche selektiert werden, zu dem Dampf.

7. Das Verfahren nach Anspruch 6, wobei das Bereitstellen einer Selektion eines oder mehrerer der Additive umfasst: Bereitstellen einer selektiven Ausrichtung einer Venturidüse (956e) zu einem oder mehreren Kanälen (926e), welche jeweils in Fluidverbindung mit einem einer Mehrzahl von Additivreservoirs (954e) stehen.

8. Das Verfahren nach Anspruch 6 oder 7, wobei das Hinzufügen des einen oder der mehreren selektierten Additive zu dem Dampf umfasst: Aktivieren mindestens eines Kristalloszillators (968f), und wobei optional das Erzeugen des Dampfes mit mindestens einem Zerstäuber (910) umfasst: Bereitstellen einer Selektion eines oder mehrerer einer Mehrzahl von Zerstäubern (910), und optional ferner umfassend: Ändern einer Position der Zerstäuber (910) in Bezug auf einen Luftströmungspfad durch die Aerosolabgabevorrichtung (700) hindurch; und
Leiten eines elektrischen Stroms zu dem einen oder den mehreren der selektierten Zerstäuber (910), um einen Dampf zu erzeugen.

## Revendications

1. Dispositif de distribution d'aérosol (700), comprenant :
au moins un atomiseur (910) configuré pour produire une vapeur à partir d'une composition de précurseur d'aérosol ; et
un sélecteur d'additifs (928) configuré pour permettre la sélection d'un ou de plusieurs d'une pluralité d'additifs ajoutés à la vapeur,
dans lequel le sélecteur d'additifs (928) comprend :
un lit de solides (940), et
un ou plusieurs éléments chauffants d'additifs (950c) configurés pour chauffer de manière sélective une ou plusieurs parties du lit de solides (940).

2. Dispositif de distribution d'aérosol selon la revendication 1, dans lequel le lit de solides (940) est positionné en aval de l'au moins un atomiseur (910) en termes d'un trajet de flux d'air, et facultativement dans lequel le lit de solides (940) comprend une pluralité de solides en plastique chargés de parfum, et facultativement dans lequel le lit de solides (940) comprend une pluralité de compartiments (942c) séparés par une ou plusieurs cloisons (946c), et comprenant en outre facultativement un élément de direction de flux (908b), le sélecteur d'additifs (928) étant configuré pour aligner de manière sélective l'élément de direction de flux (908b) avec un ou plusieurs des compartiments (942c).

3. Dispositif de distribution d'aérosol selon la revendication 1 ou 2, dans lequel l'au moins un atomiseur (910) comprend un tube de Venturi (956e), et
dans lequel le sélecteur d'additifs (928) comprend une pluralité de canaux (926e) configurés chacun pour être en communication fluidique avec l'un d'une pluralité de réservoirs d'additifs (954e) et pouvant être configurés de manière sélective pour être en communication fluidique avec le tube de Venturi (956e).

4. Dispositif de distribution d'aérosol selon l'une quelconque des revendications 1 à 3, dans lequel le sélecteur d'additifs (928) comprend au moins un oscillateur à quartz (968f).

5. Dispositif de distribution d'aérosol selon l'une quelconque des revendications 1 à 4, dans lequel l'au moins un atomiseur (910) comporte une pluralité d'atomiseurs (910) configurés chacun pour être en communication fluidique avec un réservoir respectif parmi une pluralité de réservoirs (954e), et comprenant en outre facultativement un sélecteur d'atomiseurs (628) configuré pour :
permettre la sélection d'un ou de plusieurs des atomiseurs (910) vers lequel un courant électrique est dirigé pour produire la vapeur à partir de ceux-ci ; et
modifier une position des atomiseurs (910) par rapport à un trajet de flux d'air à travers le dispositif de distribution d'aérosol (700).

6. Méthode de production de vapeur avec un dispositif de distribution d'aérosol (700), la méthode comprenant :
la formation d'un ou de plusieurs d'une pluralité d'additifs à partir d'un lit de solides (940) ;
le fait de permettre la sélection des un ou plusieurs d'une pluralité d'additifs ;
le chauffage sélectif d'une ou de plusieurs parties du lit de solides (940) pour libérer les un ou plusieurs additifs sélectionnés parmi la pluralité d'additifs du lit de solides (940) ;
la production d'une vapeur avec au moins un atomiseur (910) à partir d'une composition de précurseur d'aérosol ; et
l'ajout des un ou plusieurs additifs libérés parmi la pluralité d'additifs sélectionnés à la vapeur.

7. Méthode selon la revendication 6, dans laquelle le fait de permettre la sélection d'un ou de plusieurs des additifs comprend le fait de permettre un alignement sélectif d'un tube de Venturi (956e) avec un ou plusieurs canaux (926e) respectivement en communication fluidique avec l'un d'une pluralité de réservoirs d'additifs (954e).

8. Méthode selon la revendication 6 ou 7, dans laquelle l'ajout des un ou plusieurs additifs sélectionnés à la vapeur comprend l'activation d'au moins un oscillateur à quartz (968f), et facultativement dans lequel la production de la vapeur avec au moins un atomiseur (910) comprend le fait de permettre la sélection d'un ou de plusieurs d'une pluralité d'atomiseurs (910), et comprenant en outre facultativement la modification d'une position des atomiseurs (910) par rapport à un trajet de flux d'air à travers le dispositif de distribution d'aérosol (700) ; et
la direction d'un courant électrique vers les un ou plusieurs des atomiseurs (910) sélectionnés pour produire une vapeur.
